# EUROPEAN PATENT SPECIFICATION

(11) **EP 2 007 388 B1**
(45) Date of publication and mention of the grant of the patent: **05.06.2013**
(21) Application number: 07754711.5
(22) Date of filing: 02.04.2007
(51) Int. Cl.: A61K 31/485, A61P 25/00

(54) **OPIOPATHIES**
OPIOPATHIEN
OPIOPATHIES

(30) Priority: 03.04.2006 US 395242
(43) Date of publication of application: 31.12.2008
(73) Proprietor: Brooks-Korn, Howard, Millbrae CA 94030 (US)
(72) Inventor: Brooks-Korn, Howard, Millbrae CA 94030 (US)
(74) Representative: Glawe, Delfs, Moll
(86) International application number: PCT/US2007/008228
(87) International publication number: WO 2007/117409

(56) References cited:
- EP-A- 1 293 195
- US-A- 4 816 586
- US-A1- 2003 166 670
- US-B1- 6 586 443
- US-B2- 6 451 806

## Description

### FIELD OF THE INVENTION

The invention is set out in the appended claims and relates to a formulation for veterinary use and a veterinary product. The embodiments of the description which do not fall within the scope of said claims are provided for illustrative purposes only and do not form part of the present invention. This description relates to neuropathic, polyneuropathic, neurologic and neurogenic ailments typically characterized by paresis/paralysis. These ailments can involve an abnormal concentration of one or more endogenous opioids, or the blockade, underexpression or overexpression of one or more opioid receptors. In that regard, the description encompasses therapeutic uses, methods and compositions employing opiates and/or their receptors. In particular, the description relates to certain laboratory testing methods, clinical testing methods, research and development methods, business methods, methods of treatment, novel therapeutic uses, and human and veterinary pharmaceutical dosage forms, dosing regimens and formulations, especially those pertaining to opiopathy (particularly hypo-opiopathy).

### BACKGROUND OF THE INVENTION

Interest in the opioids and their receptors has largely focused on their analgesic use for the treatment of pain. A secondary medicinal focus has centered on uses such as cough suppression, treatment of diarrhea and sedation without loss of consciousness. The identification and understanding of opioid side effects (such as constipation, emesis, cardiac and respiratory depression, inducement of euphoria and addictiveness) has also been the subject of much research.

While some reports have noted the role of endogenous opioids as neuromuscular transmitters, these compounds' potential for abuse and their status as controlled substances are believed to have given rise to significant bias against the opioid drug class as a whole, dissuading the further elucidation of their properties and the development of active pharmaceutical agents within the drug class. In particular, the role played by endogenous opioids in maintaining normal body functions and the impact of abnormal endogenous opioid concentration has remained unassociated with the etiology and/or pathophysiology of human or animal ailments. This and the therapeutic potential of opiates for treating such ailments has for all practical purposes been overlooked until the present and my below-referenced prior patent applications.

My prior application (US03/0166670-A1; Serial No. 10/367,386) discloses the use of opiates for treatment of centrally and peripherally mediated neuropathies, polyneuropathies, disorders and syndromes including but not limited to lingual, pharyngeal, laryngeal, esophageal, urinary bladder sphincter, lumbar and lumbo-sacral spine, pelvis and pelvic-limb paresis/paralysis. This earlier application reports the successful reversal of paresis/paralysis in such disorders and syndromes, particularly by administering immediate or sustained release pharmaceutical formulations of hydrocodone; oxycodone and morphine sulfate, and teaches the similar use of other opiates. The present application further elucidates these teachings, particularly in view of the premise that abnormal concentrations of one or more endogenous opioids, or the blockade, underexpression or overexpression of one or more opioid receptors, can represent an underlying etiology of an ailment requiring treatment, but for which only palliative care has previously been available. This earlier application also did not teach the need for a diversion-resistant formulation that can be readily administered to veterinary subjects while dissuading use by humans. US 6,586,443 is addressed to treating multiple sclerosis and/or herpes by administering a low dose of an opioid antagonist such as naltrexone or naloxone. US 6,451,806 is addressed to co-administering an opioid antagonist with an opioid, such as oxycodone, in order to avoid opioid side effects while providing effective treatment of pain. EP 1 293 195 A1 is addressed to preventing the abuse by chewing or inhaling, of solid oral human pharmaceutical dosage forms that are designed to be swallowed whole. The objective is accomplished by the use of an *aversive agent* such as respiratory irritants, bitter substances and sour substances that create an aversive response in a person taking the formulation by chewing or inhaling.

### OVERVIEW AND AILMENTS OF THE MAMMALIAN NERVOUS SYSTEM

The mammalian nervous system is comprised of the Central and Peripheral Nervous Systems. The Central Nervous System ("CNS") is comprised of the brain and its functional components. The Peripheral Nervous System ("PNS") is comprised of all the cranial and spinal nerves and their functional components. Paired cranial and spinal nerves provide the means of communication between the brain, the spinal cord and the rest of the body, acting through a complex series of dynamically balanced intracellular chemical reactions.

**Terminology.** In ailments of the nervous system, when the cause originates from outside the nervous system the disorder is termed **"neurologic,"** and when the cause originates from within the nervous system it is termed "neurogenic." When a group of neurologic or neurogenic signs or symptoms are recognized together as a single ailment, it is referred to as a "disorder". When two or more disorders (with their attendant signs and symptoms) are recognized as part of a larger neurologic or neurogenic disease state, it is referred to as a "syndrome". When the clinical signs associated with a disorder or syndrome are the result of the dysfunction of a single nerve it is referred to as a **"neuropathy."** When the clinical signs associated with an ailment are the result of the dysfunction of two or more individual nerves it is referred to as a "**polyneuropathy**." The dysfunctioning nerves in a polyneuropathy can be located in either the CNS, the PNS, or in both systems simultaneously.

Ailments involving the CNS, PNS or both systems together impact the area(s) of the body normally innervated by that system or systems. In certain ailments treated according to the present description the area impacted by the nervous system dysfunction is muscle tissue, resulting in a partial or total loss of muscular function, and the ailment is called a "neuromyopathy." These ailments are observed individually or as part of a larger neurologic and/or neurogenic syndrome, and can be inherited or acquired. When partial function remains in the innervated muscle tissue, it is termed "paresis." When no function remains in the innervated muscle tissue, it is termed "paralysis." "Paresis/paralysis" or "P" is defined for purposes of the present invention as partial or total loss of function in innervated muscle tissue.

**Lingual paresis/paralysis** ("LiP") affects the ability of an individual to prehend food, pass a food bolus to the back of the pharynx and interferes with the individual's ability to swallow food, saliva or water; the resulting disorder is known as "Oral or Lingual Dysphagia." If the individual's nutritional needs are not effectively addressed, death can occur as the result of the body's physical deterioration and eventual organ shutdown from the prolonged effects of dehydration, malnutrition and eventual starvation. LiP can also obstruct the upper airway, potentially leading to aspiration pneumonia and/or suffocation. To date there is no known cure for LiP. The focus of therapy remains on strategies to insure an adequate dietary intake of food and water, maintaining an open airway, management of effective oral hygiene and treating the consequences of aspiration pneumonia.

**Pharyngeal paresis/paralysis ("PhP")** can disrupt normal gag and/or swallow reflexes resulting in the ineffective swallowing of food and water, can lead to aspiration pneumonia (because the opening into the trachea is ineffectively covered during swallowing), can allow regurgitation of food or fluid back up into the oral and nasal cavities and can impair the normal passage of air into the trachea; the resulting disorder is known as "Pharyngeal Dysphagia." If an individual's nutritional and airway needs are not adequately addressed, death can occur as the result of complications of starvation, aspiration pneumonia and/or suffocation. To date there is no known cure for PhP. The focus of therapy remains on strategies to insure adequate nutritional intake while addressing continual problems associated with fluid and food aspiration into the lungs and maintaining an open airway.

**Laryngeal paresis/paralysis ("LaP")** can impair one's ability to phonate, can cause an upper airway obstruction severely decreasing airflow into the lungs, and can allow aspiration of food and fluid into the trachea (because the arytenoids fail to effectively close over the tracheal opening during swallowing); the resulting disorder in dogs and cats is known as "Recurrent Laryngeal Nerve Paralysis." A comparable equine disorder is called "roaring." If the medical affects of laryngeal paresis/paralysis are not effectively dealt with, death can occur as the result of complications from aspiration pneumonia, respiratory failure and finally cardiac arrest. To date there is no known cure for LaP. The focus of therapy remains on strategies to maintain an open and adequate airway into the trachea allowing sufficient oxygen to reach the lungs and on strategies to deal with aspiration pneumonia and its consequences. Historically, when respiratory difficulty attributed to LaP presented contemporaneously with pelvic and/or spinal paresis/paralysis, the ailment was referred to as "Laryngeal Paralysis - Polyneuropathy Complex."

**Esophageal paresis/paralysis ("EP")** involves a loss of normal peristaltic movement of food down the esophagus and into the stomach, and can result in retention of masticated food and fluid in the esophagus. Such retention of food and fluid causes an inflammatory response that can lead to retention esophagitis, triggering regurgitation of esophageal contents into the oral and nasal pharynx, and can allow aspiration of the regurgitated esophageal contents into the lungs. The most common disorder associated with EP is known as "**Megaesophagus**." Death from Megaesophagus can ensue from the long-term effects of starvation, as a result of complications of "Retention Esophagitis", and/or from the secondary complications of aspiration pneumonia. To date there is no known cure for EP. The focus of therapy remains on strategies to passively allow masticated food and fluid to flow from the oral pharynx into the stomach, and on medical strategies for treating the resultant esophagitis including neutralizing the affects of differing chemical compositions on mucosal surfaces when positional aids fail to prevent the passive backward movement of foodstuffs into the oral/nasal pharynx, and treating the effects of aspiration pneumonia if they occur.

Neurogenic urinary bladder sphincter paresis/paralysis ("NUBSP") can result in intermittent or continual leaking of urine out of the bladder; the resulting disorder is known as **"Neurogenic Urinary Bladder Sphincter Incontinence."** The leaking urine's pathway or site of accumulation determines the symptoms associated with this incontinence. Urethritis, Cystitis, Nephritis, Vaginitis, Perivulvar and Vulvar Vaginitis and Urine Scald Dermatitis are some of the secondary consequences associated with NUBSP. Other forms of urinary bladder incontinence differ significantly from NUBSP. For example, urinary bladder incontinence can also result from cancer of the sphincter, from the lodging of a foreign body in the sphincter (such as with cysto uroliths, aka bladder stones), from overload incontinence (where consuming too much liquid causes the sphincter to fail when it becomes unable to hold back abnormally large volumes of urine) and from urge incontinence (where the patient suffers from the sensation of needing to urinate when the bladder is not full, even though there is no pathology in the bladder or the bladder sphincter). To date there is no known cure for NUBSP. The focus of therapy remains on strategies to control urine leakage (e.g., Urinary Bladder Suspension Surgery, which is available in the few exceptional cases where correcting an anatomical defect would treat the incontinence) or to absorb the leaking urine (using absorbent sanitary pads or undergarments), to treat primary and secondary areas of inflammation or infection, and to keep the leaking and leaked-on areas as clean, dry, and sanitary as possible.

Lumbar and lumbo-sacral spine **paresis/paralysis** ("LLSP") can cause progressive loss of function of the skeletal muscles over the lumbar and lumbo-sacral spine, which presents visually as atrophy and weakness in these areas. As the disorder progresses, it becomes increasingly more difficult to use the back in even the most basic of functions such as in bending, straightening and turning the upper torso. To date there is no known cure for LLSP. The focus of therapy remains on strategies to assist one with ambulation, sitting, standing and reclining, such as providing specially designed walkers, canes, rails, ramps, power assisted lifts, etc.

**Pelvis and pelvic limb paresis/paralysis ("PPLP")** causes progressive loss of function and eventual paralysis of the muscles over the pelvis and pelvic limbs, which presents visually as atrophy and weakness in these areas. Progressive loss of muscle tone and strength in the pelvis and pelvic limbs make even rudimentary functions such as standing, sitting, rising, and ambulating almost impossible without some sort of external assistance. To date there is no known cure for PPLP. The focus of therapy remains on strategies for assisted movements when standing, walking or sitting, such as providing specially designed walkers, canes, crutches and carts. Eventually any function requiring muscular movement or strength below the waist will fail.

**Neuropathic atrophy** entails the wasting of muscle following a protracted period of abnormal innervation. Related human ailments include spinal muscular atrophy (SMA) and spinal muscular atrophy with respiratory distress type 1 (SMARD1). As discussed on the National Institute of Neurological Disorders and Stroke's Spinal Muscular Atrophy Information Page http://www.nindsnih.gov/disorders/sma/sma.htm (from which the following descriptions are taken) the spinal muscular atrophies are all autosomal recessive diseases. SMA type I (also called Werdning-Hoffmann disease) is evident before birth or within the first moments of life. It is a disease reportedly caused by mutations in the telomeric survival motor neuron gene involving neurogenic atrophy primarily in proximal muscle groups; symptoms include floppiness of limbs and trunk, feeble movements of the arms and legs, swallowing and feeding difficulties and impaired breathing. Affected children never sit or stand and usually die before the age of two. SMA type II usually begins between 3 and 15 months of age; symptoms include respiratory problems, floppy limbs, decreased or absent deep tendon reflexes, and twitching of arm, leg, or tongue muscles. These children may learn to sit, but will never be able to stand or walk; life expectancy varies. SMA type III (also called Kugelberg-Welander disease) symptoms appear between 2 and 17 years of age, and include abnormal manner of walking; difficulty running, climbing steps, or rising from a chair; and slight tremor of the fingers. Progressive spinobulbar muscular atrophy (or Kennedy syndrome) can occur between 15 and 60 years of age. Symptoms include weakness of muscles in the tongue and face, difficulty swallowing, speech impairment, and excessive development of mammary glands in males.

SMARD1 is an autosomal recessive motor neuron disease that affects infants, presenting with respiratory distress due to diaphragmatic paralysis and progressive (predominantly distal lower limb) muscle weakness. SMARD1 reportedly results from mutations in the gene encoding immunoglobulin µ-binding protein 2 (IGHMBP2), putatively a member of the superfamily 1 RNA helicases, which are involved for example in transcription, translation, splicing, nuclear export, ribosome biogenesis and nonsense-mediated m-RNA decay among other protein-protein interactions. An experimental model of SMARD1 has been described in *nmd* mutant mice. (See, e.g., Maddatu, T., et al., Human Molecular Genetics, 2004, Vol. 13, No. 11 1105-1115 and Grohmann, K., et al., Human Molecular Genetics, 2004, Vol. 13, No. 18 2031-2042.)

**Sarcopenia** is described as the age-related loss of skeletal muscle mass and function (as characterized by strength and fatigability) starting as early as the fourth decade of life in humans. Reduced muscle strength in the elderly is a major cause for their increased prevalence of disability such as the inability to walk and falling. Distinct muscle changes have been reported to be associated with sarcopenia, including a decrease in type 2 muscle fibers, mixed muscle protein synthesis, myosin heavy chain synthesis, and mitochondrial protein synthesis. (See, e.g., Karakelides, H. et al., Curr. Top. Dev. Biol., 2005;68:123-48.)

**Pain** and the treatment thereof, is discussed in Goodman & Gilman's The Pharmacological Basis of Therapeutics, 11th Edition, Chapter 21 regarding the action of analgesic agents. There is a "distinction between *pain as a specific sensation,* subserved by distinct neurophysiological structures, and *pain* as *suffering* (the original sensation plus the reactions evoked by the sensation). It generally is agreed that all types of painful experiences, whether produced experimentally or occurring clinically as a result of pathology, include the original sensation and the reaction to that sensation. It also is important to distinguish between pain caused by stimulation of nociceptive receptors and transmitted over intact neural pathways *(nociceptive* pain) and pain that is caused by damage to neural structures, often involving neural supersensitivity *(neuropathic* pain). Although nociceptive pain usually is responsive to opioid analgesics, neuropathic pain typically responds poorly to opioid analgesics and may require higher doses of drug (citation omitted)."

Ailments such as the foregoing are often progressive in nature and can eventually result in permanent dysfunction of the particular organ or area of the body involved. As only palliative treatment is currently available to those suffering from such debilitating ailments, there exists a considerable need for better therapeutic alternatives. It should be noted that the present invention does not pertain to the treatment of morphine-induced respiratory failure/depression/paralysis, or to the treatment of botulism or its sequellae, or to treating like ailments resulting from an exogenous cause, injury or toxin.

### THE OPIOIDS

Endogenous opioid peptides serve as hormones and as neuromodulators. They fall within three distinct families: the enkephalins, endorphins and dynorphins, respectively being derived *in* situ from the distinct precursors preproenkephalin, pro-opiomelanocortin ("POMC") and preprodynorphin, which are in turn encoded by three corresponding genes. They range in size from 5 to 31 residues, share a common amino-terminal sequence (the opioid motif), and vary over their C-terminal ends. A more recently discovered neuropeptide system having a high degree of sequence identity to the opioid peptides has been called the *nociceptin*/*orphanin FQ* (or "N/OFQ") system. Endogenous opioid peptides that serve as hormones are secreted into the circulation by the producing glands and delivered to a variety of distant target tissues where they induce a response. All three types of opioid peptides are found in the pituitary, the adrenal glands, the hypothalamus and the brain stem, as well as in many organ tissues throughout the body including the heart, pancreas, placenta, kidneys and gastrointestinal organs. Endogenous opioid peptides that serve as neuromodulators are produced and secreted by nerve cells and act in the brain and spinal cord to modulate the actions of other neurotransmitters.

The enkephalins include: leu-enkephalin, met-enkephalin, met-enkephalin-Arg-Phe, met-enkephalin-Arg-Gly-Leu, and a series of peptides containing met-enkephalin at the N-terminus including peptide E and peptide F. Pro-enkephalin peptides are found in areas of the CNS that are presumed related to the perception of pain, modulation of behavior, modulation of motor control, regulation of the autonomic nervous system and neuroendocrinological functions. These peptides are also found in the adrenal medulla and in nerve plexuses and exocrine glands of the stomach and intestine. Most enkephalin-containing neurons have short axons, indicating that enkephalins act close to their points of synthesis. The endorphins include: α-endorphin, β-endorphin and γ-endorphin. Their precursor POMC is produced by cells located mainly within the CNS, having a distribution that corresponds to areas of the human brain where electrical stimulation can relieve pain. Neurons containing β-endorphin can be found predominantly in the hypothalamus and in the nucleus of the solitary tract, a region of the brain stem. Peptides from POMC occur in the anterior and intermediate lobes of the pituitary and also in pancreatic islet cells. POMC also contains the primary sequences for adrenocorticotrophic hormone ("ACTH"), for α-melanocyte-stimulating hormone ("α-MSH") and for β-lipotropin ("β-LPH"); its production is stimulated by Corticotropin Releasing Hormone (CRH); tissue-specific cleavage is performed by precursor convertases PC1 and/or PC2±7B2. The dynorphins include: dynorphin A, dynorphin B, b-neoendorphin, and smaller peptides such as dynorphin A1-8, dynorphin A1-13 and dynorphin A1-17. Neurons containing peptides from preprodynorphin are diffusely distributed in the brain, e.g., in the hypothalamus. Neurons containing β-endorphin or dynorphins have long axons that extend to distant brain regions as well as to the pituitary gland, brain stem and spinal cord, indicating that the peptides act distant to their points of synthesis.

Three major types of opioid receptors have been identified: mu (µ), delta (δ) and kappa (κ); there is also an N/OFQ receptor. They all belong to the G protein-coupled receptor (GPCR) family. Each type of receptor is believed to have multiple sub-types. These receptors have unique anatomical distributions in the brain, spinal cord and the periphery (as determined by autoradiographic techniques). Endorphins are believed generally associated with δ receptors, while the dynorphins primarily associate with κ receptors; the latter exhibiting the greatest selectivity across endogenous ligands. Notwithstanding such selectivity, there is significant "cross-talk" between peptide and receptor types. A given opioid peptide can interact with more than one type of opioid receptor varying, e.g,. in a concentration-dependent manner.

In the modulation of neurotransmitters, endogenous opioid peptides are often released together with other neurotransmitter molecules in the brain, pituitary gland, adrenal gland, and by single neurons in the CNS and PNS. The function of co-releasing peptide neurotransmitter pairs has yet to be completely elucidated, but evidence suggests that the opioid peptides can alter the release rates of other classic neurotransmitters, for example, inhibiting release of acetylcholine, dopamine and norepinephrine, or modulating serotonin and gamma-aminobutyric acid release either up or down. These neurotransmitters are directly involved in transmitter-gated ion channels transmitting nerve impulses that stimulate muscle cells to contract. The impact of such modulation can ultimately result in increased or decreased neurotransmission, for example, depending upon whether the neuron being modulated is an inhibitory neuron. Opioid peptides are also reported to make their target neurons more difficult to excite by increasing the voltage difference that exists between the inside and outside of the cell, hyperpolarizing the neurons and thereby reducing firing rates and neurotransmitter release.

Opiates are chemically classified as alkaloid compounds. The prototypic opiate, morphine, was first isolated from the opium poppy *(papaver somniferum)* in the early nineteenth century. The opiates can be broadly divided into five distinct chemical groups: phenanthrene, benzylisoquninoline, tetrahydroisoquinoline, cryptopine, and miscellaneous (Remington's Pharmaceutical Sciences 433, 1975). Therapeutically useful drugs have been primarily derived from the phenanthrene and benzylisoquinoline classes. The principal phenanthrenes are morphine, codeine, and thebaine. The principal benzylisoquinolines are papaverine and noscapine.

The most common use of opiates in today's prescription market is for their analgesic properties. Opiates, like the endogenous opioid peptides, produce their effects via binding to the various types of opioid receptors throughout the CNS and PNS; a given opiate can bind with one or more types of receptor. Opiates reportedly act as analgesics by elevating the pain threshold and altering the psychological response to pain. Pharmacologic effects vary among opiates, depending on the receptor, its location in the body, and the type of interaction between the opiate and the receptor.

Although the primary pharmacologic effects of most opiates as used today are analgesia, anti-tussive and sedation without loss of consciousness (along with inappropriate use for inducing euphoria) the pharmacologic affects of opiates, like the endogenous opioids, extend beyond the control of pain. One opiate, apomorphine, directly stimulates the chemoreceptor trigger zone in the brain, triggering an emetic or vomiting response (which can be helpful in an emergency situation where one wants to stimulate emesis) whereas butorphanol (another opiate) has been used as an *anti*-emetic, to help control vomiting induced by the chemotherapeutic agent Cisplatin (Schurig, *et* al., 1982). Additional gastrointestinal effects noted in response to the administration of opiates include: increase or decrease in the amount of hydrochloric acid secreted into the stomach, and increase in tone in the antral portion of the stomach and upper duodenum (resting segmental tone is increased, markedly decreasing the propulsive movement of the intestinal contents, which is helpful in treating upper intestinal diarrhea, but can lead to the common opiate-related side effect of constipation if diarrhea is not present).

Wider ranging prospective uses for opiates have also been proposed. One study of endogenous opioids (Khan, et al., "Effect of β-endorphin on the contractile responses in mouse skeletal muscle," Muscle & Nerve, 18:1250-1256, 1995) tested several endogenous opioid receptor-specific agonists for their actions on skeletal muscle. The study concluded that "specific opioid agonists may have clinical application in the treatment of neuromuscular diseases such as myasthenia gravis in which the defect is manifest at the neuromuscular junction." Noting that current treatments with reversible cholinesterase inhibitors such as pyridostigmine are non-specific and lead to side effects due to actions at muscarinic sites, the possibility of using opioids to avoid such side effects was conditionally mentioned "if an agonist which acts on a specific subtype of receptor can be developed." US Patent No. 6,723,343 discusses the use of a sugar substitute-containing formulation of a tramadol salt for treating pain, urinary incontinence, coughs, inflammatory and allergic reactions, depression, drug and alcohol abuse, gastritis, diarrhea, cardiovascular disease, respiratory disease, mental illness and epilepsy. WO O2/060445 describe a series of κ opioid receptor-specific compounds for treating disease states ameliorated by binding opioid receptors, including as: "cytostatic agents, as antimigraine agents, as immunomodulators, as immunosuppressives, as antiarthritic agents, as antiallergic agents, as virucides, to treat diarrhea, as antipsychotics, as antischizophrenics, as antidepressants, as uropathic agents, as antitussives, as antiaddictive agents, as anti-smoking agents, to treat alcoholism, as hypotensive agents, to treat and/or prevent paralysis resulting from traumatic ischemia, general neuroprotection against ischemic trauma, as adjuncts to nerve growth factor treatment of hyperalgesia and nerve grafts, as anti-diuretics, as stimulants, as anti-convulsants, or to treat obesity, additionally mentioning treatment of dyskinesia associated with L-dopa treatment in Parkinson's disease.

Notwithstanding the foregoing, it has remained unknown until the present application that a group of mammalian ailments can be attributed to abnormal endogenous opioid levels and can be treated by administration of one or more anti-opiopathic active agents.

### SUMMARY OF THE INVENTION

The invention is set out in the appended claims and relates to a formulation for veterinary use and a veterinary product. The embodiments of the description which do not fall within the scope of said claims are provided for illustrative purposes only and do not form part of the present invention. If you were in a physician's office in 2006 it would appear fairly routine to hear a statement such as: "The test results show that your thyroid hormone level is too low, so we're going to give you a medicine that provides what you're missing. You should be feeling well in no time." Endogenous opioids perform numerous functions in a healthy body, far beyond the quieting of nociceptive pain. It has now been observed that abnormal concentrations of these naturally occurring substances (i.e., "opiopathy") can manifest in the form of ailments that have to date been completely unassociated with opioids or their endogenous concentrations. Opiopathies are believed to be so pervasive that in the future it will become common in a physician's office to hear statements such as: "The test results show that one of your opioid levels is too low, so we're going to give you a medicine that provides what you're missing. You should be feeling well in no time."

The present description provides novel methods for classifying, diagnosing and/or treating a group of human and veterinary ailments involving endogenous opioid concentrations. Also provided is a novel use for an existing class of compounds, the opioids, to treat opiopathic ailments, particularly paresis/paralysis, pseudo-atrophy and/or opiopathic pain, and in the manufacture of pharmaceutical and veterinary formulations therefor. The description also relates to neuropathic, polyneuropathic, neurologic and neurogenic ailments typically characterized by paresis/paralysis. These ailments can involve an abnormal concentration of one or more endogenous opioids, or the blockade, underexpression or overexpression of one or more opioid receptors. In that regard, the description encompasses therapeutic uses, methods and compositions employing opiates and/or their receptors. In particular, the description relates to certain laboratory testing methods, clinical testing methods, research and development methods, business methods, methods of treatment, novel therapeutic uses, and human and veterinary pharmaceutical dosage forms, dosing regimens and formulations, especially those pertaining to opiopathy (particularly hypo-opiopathy).

One aspect of the present description provides methods of treatment, particularly a method for treating an opiopathy by administering to a subject in need thereof an effective amount of an anti-opiopathic active agent. The opiopathy treated in such method can involve an abnormal level of an endogenous opioid. It can be a hypo-opiopathy characterized by deficiency of an endogenous opioid. The anti-opiopathic active agent employed in these methods can be an exogenous equivalent or replacement for such an endogenous opioid, or be selected as having the same opioid receptor type specificity as the endogenous opioid.

The opiopathies treated in these methods can involve any of the following individual ailments, groups of ailments or sub-groups thereof:
- paresis/paralysis, pseudo-atrophy, opiopathic pain, immune surveillance, tumor surveillance, behavior modulation, neuromuscular modulation or neuroendocrine modulation;
- paresis/paralysis, pseudo-atrophy, opiopathic pain, immune surveillance, tumor surveillance or neuroendocrine modulation:
- paresis/paralysis, pseudo-atrophy or opiopathic pain;
- paresis/paralysis or pseudo-atrophy;
- Upper Respiratory Obstructive Syndrome or Opioid-responsive Polyneuropathic Syndrome;
- lingual, pharyngeal, laryngeal, esophageal, neurogenic urinary bladder sphincter, lumbar and lumbo-sacral spine, and pelvis and pelvic limb paresis/paralysis;
- opioid-responsive neurogenic urinary bladder sphincter paresis/paralysis;
- cardiomyopathy, centrally mediated depression, congestive heart failure, or paralytic Intestinal ileus;
- Multiple Autonomic Nervous System Dysfunction, Multiple Sclerosis, Myasthenia Gravis, Parkinson's Disease, Post-Polio Syndrome or ALS; and
- Multiple Autonomic Nervous System Dysfunction, Multiple Sclerosis, Parkinson's Disease, Post-Polio Syndrome or ALS.

The opiopathies and methods of treatment can likewise involve any of the following:
- pseudo-atrophy, where the treatment results in a rapid return of muscle function and tone as compared to treatment of atrophy;
- Multiple Sclerosis, Parkinson's Disease or ALS, where the anti-opiopathic active agent includes an opiate agonist and an opioid antagonist;
- Multiple Sclerosis, where the anti-opiopathic active agent is administered in an amount sufficient to normalize neuronal and neuromuscular transmission, and to down-regulate IL-12;
- Multiple Sclerosis, where the anti-opiopathic active agent is hydrocodone or oxycodone, administered in an amount sufficient to treat emotional incontinence;
- Multiple Sclerosis, where the anti-opiopathic active agent is hydrocodone, administered in an amount sufficient to treat emotional incontinence; and
- Myasthenia Gravis, where the anti-opiopathic active agent (e.g., oxycodone hydrochloride) is a very low dose of an immediate release formulation.

The subject treated in any of the foregoing methods is a mammal, and can be a human or a non-human mammal. The subject can be a human. The subject can be a dog.

The anti-opiopathic active agent employed in any of the methods can be is any individual member, group or sub-group of the following:
- morphine, codeine, thebaine, papaverine, noscapine, hydromorphone, metapon, oxymorphone, levorphanol, hydrocodone, oxycodone, tramadol, nalorphine, naloxone, naltrexone, meperidine, a meperidine congener, methadone, a methadone congener, levorphanol, a levorphanol congener, phenazocine, propoxyphene, ethoheptazine, or a pharmaceutically or veterinarily acceptable salt thereof;
- morphine, codeine, hydromorphone, hydrocodone, oxycodone, naloxone, naltrexone or a pharmaceutically or veterinarily acceptable salt thereof; and
- morphine, oxycodone, or a pharmaceutically or veterinarily acceptable salt thereof.
The anti-opiopathic active agent employed in these methods can also include an opioid agonist (preferably morphine, oxycodone, tramadol or hydrocodone) and an opioid antagonist (preferably naltrexone). Alternatively, the anti-opiopathic active agent employed in any of the foregoing methods can be an opioid peptide precursor or a vector for introducing a recombinant gene to modulate *in-situ* opioid or opioid receptor expression.

Identifying the proper anti-opiopathic active agent and dose for a subject treated in a method of the description can be accomplished by the following steps: (a) administering an initial dosage of an anti-opiopathic active agent for an initial period of time, (b) determining whether that dosage provides effective treatment for the subject, (c) if the dosage is determined to provide effective treatment, continuing to administer the anti-opiopathic active agent at the initial dosage, or (d) if the initial dosage is determined not to provide effective treatment, increasing the dosage by a factor ranging from about 1.25 to 2.0 at which dosage the anti-opiopathic active agent is administered for a subsequent period of time, and (e) repeating steps (b) and (c) or (d) until effective treatment is provided, or if the subject's maximum tolerated dosage is reached changing to a different anti-opiopathic active agent or discontinuing treatment. The initial and subsequent periods of time for each dose escalation step are about 2 to 14 days. A preferred dose escalation is 1.5 times the amount of the previous dosage.

Still another aspect of the description provides uses of an anti-opiopathic active agents and/or opioids in the manufacture of a medicament for treating any one or more of the following ailments: opiopathy, pseudo-atrophy, Upper Respiratory Obstructive Syndrome, Opioid-responsive Polyneuropathic Syndrome, and Opioid-responsive Neurogenic Urinary Bladder Sphincter Paresis/paralysis.

Also provided are pharmaceutical or veterinary formulations for treating an opiopathy comprising an anti-opiopathic active agent and a pharmaceutically or veterinarily accepted excipient. The opiopathies treated with these formulations can involve any of the individual, groups or sub-groups of ailments, for example, as recited above in paragraphs 033 and 034. The anti-opiopathic active agent employed in such formulations can be as set forth in paragraph 036. Such formulations can include a detractant to render them unsuitable for diversion, e.g., adding capsaicin to a tablet in an amount that would not interfere with normal swallowing and absorption, but would dissuade misuse by dissolving the tablet for injection.

The veterinary formulations of the invention also include a detractant ingredient, for example, an odor, flavor, texture or other ingredient that while palatable to a non-human mammal is unacceptable to a human being.. In certain such formulations of the invention, the detractant would be considered a contaminant if included in a formulation intended for human consumption (e.g., hair, sand, insect parts or feces, treated to be non-harmful for a subject of the species for which the formulation is intended). Alternatively, the veterinary formulations of the invention can be manufactured in a dosage form that is unsuitable for human consumption, e.g., a chew bone. Veterinary products comprising such formulations can have outer packaging prominently labeled to highlight the presence of the detractant as a warning against human consumption.

A pharmaceutical or veterinary product according to the description can be used in patient familiarization and/or dose ranging with the methods and formulations of the description, including: (a) a pharmaceutical or veterinary formulation having an anti-opiopathic active agent at a starting dosage level, in a quantity sufficient for administration over an initial period of time, (b) a second such formulation having said anti-opiopathic active agent at an incrementally higher dosage level wherein the dosage is increased by a factor ranging from about 1.25 to 2.0, in a quantity sufficient for administration over a subsequent period of time, and (c) instructions for administration of the anti-opiopathic active agent and determination of therapeutically effective and maximum tolerated doses. The first and second formulations can be separately packaged and/or labeled to facilitate distinguishing therebetween and completion of dosing during each such period of time.

Another aspect of the description provides methods for diagnosis (and in some cases diagnosis and treatment) of a subject suspected of having an opiopathic ailment. One such method entails determining whether any of the subject's endogenous opioid levels are abnormal, and upon identifying an endogenous opioid level abnormality, administering to the subject a therapeutically effective amount of an anti-opiopathic active agent sufficient to treat the opiopathy. Alternatively, upon identifying no endogenous opioid level abnormality, the provides for concluding that the subject does not suffer from opiopathy and evaluating alternative diagnoses and treatments.

The determination of endogenous opioid level can be made by laboratory analysis of a specimen (e.g., blood, serum, plasma, urine, synovial fluid, cerebral/spinal fluid, lymphatic fluid or a tissue biopsy) obtained from the subject, for example by ELISA. or RRA. The amounts of endogenous opioids in the specimen are evaluated to determine whether the level of any of the endogenous opioids is abnormal. Such evaluation can be made, e.g., by comparison against baseline levels obtained by similarly testing one or more normal subjects.

Alternatively, the determination of endogenous opioid level can be accomplished by a clinical analysis of the subject, for example by PET. Such PET analyses will typically entail administering a physiologically acceptable radionuclide-labeled opioligand to the subject and measuring a local level thereof. Alternatively, a series of physiologically acceptable, differentially radionuclide-labeled opioligands representative of a normal endogenous opioid distribution can be administered to the subject and local levels of the respective agents measured by PET. Such PET methods can be performed by first conducting a baseline PET scan, administering one or more of such opioligands, waiting for a period sufficient to permit opioligand localization, repeating the PET scan and comparing the results obtained against the baseline. Alternatively, the measured concentration or distribution of an endogenous opioid can be compared against a normal standard or compared against an opioligand distribution known to confirm the suspected opiopathic condition. The opioligand employed in such analyses can be an anti-opiopathic active agent specific for an opioid receptor known to be associated with the subject's suspected ailment, for example, ¹³N-radionuclide-labeled oxycodone or ¹⁵O-radionuclide-labeled oxycodone or a pharmaceutically or veterinarily acceptable salt thereof.

A veterinary diagnostic method for a subject suspected of suffering from canine UROS can be performed by holding the subject's mouth closed and determining whether the signs and symptoms of UROS remain apparent during nasal respiration; the absence of respiratory obstructive signs arid symptoms during nasal respiration in a subject that exhibits such signs during open-mouthed breathing is indicative of UROS. By way of confirmation in a subject positively tested as described immediately above, an anti-opiopathic active agent can be administered to the subject followed by repeated observation for the persistence or resolution of such signs and symptoms during open-mouthed breathing.

Another diagnostic aspect of the description provides a method for determining the opiate responsiveness profile of a subject, by co-administering to the subject a series of physiologically acceptable radionuclide-labeled anti-opiopathic active agents and measuring local levels of the respective agents by PET.

Still another assay-related aspect of the description provides a method of determining whether a test substance is an anti-opiopathic active agent, by (a) providing a test subject that overproduces or underproduces an endogenous opioid corresponding to an opiopathic characteristic other than the amount produced of such opioid; (b) observing the characteristic in the subject; (c) administering the test substance to the subject; (d) observing the characteristic in the treated subject; and (e) comparing the characteristic before and after administration of the test substance, where treatment of the characteristic corresponds to anti-opiopathic activity. The test subject can be a recombinant mouse, or the like.

Kits are also provided, for example, for use in the diagnostic methods and assays of the description including an opioligand labeled with a physiologically acceptable radionuclide. The kits can include more than one such labeled opioligand, where such opioligands representing different binding classes and/or endogenous opioids associated with an opiopathic condition and can preferably be simultaneously detected and distinguished by PET. Such opioligands can be formulated with a pharmaceutically or veterinarily acceptable excipient and provided in a dosage form suitable for administration to a test subject.

Another of the diagnostic and assay aspects of the description provides a positron emission tomography device having hardware for the *in situ* diagnosis of an opiopathy, and software to analyze the data obtained by use of the hardware (in view of baseline endogenous opioid levels) and generate a report thereon. Such report can include an identification of an endogenous opioid measured at an abnormal level or even a specific diagnostic recommendation. The methods of diagnosis, assays, kits and devices of the description can be further employed in methods of collecting opiopathic clinical data, for example, by obtaining a sample from a human or a non-human mammal exhibiting symptoms of an opiopathic ailment and determining the endogenous opioid levels of said sample.

Irrespective of the present application's proposals regarding mechanism of action, nomenclature (e.g., opiopathy) and the like, the fact remains that a group of ailments that had previously been considered untreatable, have been reproducibly treated by administering a therapeutically effective amount of an opiate. In this regard, also provided is a method for treating any of the following individual ailments, group of ailments or sub-groups thereof:: paresis/paralysis, pseudo-atrophy, Upper Respiratory Obstructive Syndrome, Opioid-responsive Polyneuropathic Syndrome, cardiomyopathy, centrally mediated depression, congestive heart failure, paralytic intestinal ileus, Multiple Autonomic Nervous System Dysfunction, Multiple Sclerosis, Myasthenia Gravis, Parkinson's Disease, Post-Polio Syndrome and ALS, by administering to a subject in need thereof an effective amount of an opiate. The ailment treated can involve any of the following individual ailments, groups of ailments or sub-groups thereof:
- Upper Respiratory Obstructive Syndrome or Opioid-responsive Polyneuropathic Syndrome;
- lingual, pharyngeal, laryngeal, esophageal, urinary bladder sphincter, lumbar and lumbo-sacral spine, and pelvis and pelvic limb paresis/paralysis;
- opioid-responsive neurogenic urinary bladder sphincter paresis/paralysis;
- cardiomyopathy, centrally mediated depression, congestive heart failure, or paralytic intestinal ileus;
- Multiple Autonomic Nervous System Dysfunction, Multiple Sclerosis, Myasthenia Gravis, Parkinson's Disease, Post-Polio Syndrome or ALS; and
- Multiple Autonomic Nervous System Dysfunction, Multiple Sclerosis, Parkinson's Disease, Post-Polio Syndrome or ALS.

The methods of treatment can also involve any of the following:
- pseudo-atrophy, where the treatment results in a rapid return of muscle function and tone as compared to treatment of atrophy;
- Multiple Sclerosis, Parkinson's Disease or ALS, where the active agent includes an opiate agonist and an opioid antagonist;
- Multiple Sclerosis, where the active agent is administered in an amount sufficient to normalize neuronal and neuromuscular transmission, and to down-regulate IL-12;
- Multiple Sclerosis, where the active agent is hydrocodone or oxycodone, administered in an amount sufficient to treat emotional incontinence;
- Multiple Sclerosis, where the active agent is hydrocodone, administered in an amount sufficient to treat emotional incontinence; and
- Myasthenia Gravis, where the active agent (e.g., oxycodone hydrochloride) is a very low dose of an immediate release formulation.

The subject treated in any of the foregoing methods is a mammal, and can be a human or a non-human mammal. The subject can be a human. The subject can be a dog.

The active agent employed in any of the methods can be is any individual member, group or sub-group of the following:
- morphine, codeine, thebaine, papaverine, noscapine, hydromorphone, metapon, oxymorphone, levorphanol, hydrocodone, oxycodone, tramadol, nalorphine, naloxone, naltrexone, meperidine, a meperidine congener, methadone, a methadone congener, levorphanol, a levorphanol congener, phenazocine, propoxyphene, ethoheptazine, or a pharmaceutically or veterinarily acceptable salt thereof;
- morphine, codeine, hydromorphone, hydrocodone, oxycodone, naloxone, naltrexone or a pharmaceutically or veterinarily acceptable salt thereof; and
- morphine, oxycodone, or a pharmaceutical or veterinarily acceptable salt thereof.
The anti-opiopathic active agent employed in these methods can also include an opioid agonist (preferably morphine, oxycodone, tramadol or hydrocodone) and an opioid antagonist (preferably naltrexone). Alternatively, the anti-opiopathic active agent employed in any of the foregoing methods can be an opioid peptide precursor or a vector for introducing a recombinant gene to modulate *in-situ* opioid or opioid receptor expression.

Identifying the proper opiate and dose for a subject treated in a method of the description can be accomplished by the following steps: (a) administering an initial dosage of the opiate for an initial period of time, (b) determining whether that dosage provides effective treatment for the subject, (c) if the dosage is determined to provide effective treatment, continuing to administer the opiate at the initial dosage, or (d) if the initial dosage is determined not to provide effective treatment, increasing the dosage by a factor ranging from about 1.25 to 2.0 at which dosage the opiate is administered for a subsequent period of time, and (e) repeating steps (b) and (c) or (d) until effective treatment is provided, or if the subject's maximum tolerated dosage is reached changing to a different opiate, or discontinuing treatment. The initial and subsequent periods of time for each dose escalation step are about 2 to 14 days. A preferred dose escalation is 1.5 times the amount of the previous dosage.

Except as otherwise specifically provided, the opiate or anti-opiopathic active agent employed in any aspect of the present description can be formulated and/or administered as a sustained release formulation.

### DETAILED DESCRIPTION OF THE INVENTION

It is to be understood that the terminology used herein is for the purpose of describing particular embodiments only, and is not intended to be limiting.

### Definitions

As used in the present specification, the following words and phrases are generally intended to have the meanings as set forth below, except to the extent that the context in which they are used indicates otherwise. The following abbreviations and terms have the indicated meanings throughout:
- EP: esophageal paresis/paralysis
- LaP: laryngeal paresis/paralysis
- LiP: lingual paresis/paralysis
- LLSP: lumbar and lumbo-sacral spine paresis/paralysis
- NUBSP: neurogenic urinary bladder sphincter paresis/paralysis
- PhP: pharyngeal paresis/paralysis
- PPLP: pelvis and pelvic limb paresis/paralysis
- P: when used at the end of a defined abbreviation means paresis/paralysis
- OR: when used at the beginning of a defined abbreviation means opioid-responsive
- ORNUBSP: opioid-responsive neurogenic urinary bladder sphincter paresis/paralysis
- ORPS: opioid-responsive polyneuropathy, syndrome
- UROS: upper respiratory obstructive syndrome

As used in the specifications and claims, the singular form is intended to include the plural unless the context clearly dictates otherwise. For example, the term "an opioid compound" encompasses one or more opioids, as well as mixtures thereof.

The terms "anti-opiopathic agent", "anti-opiopathic active agent" and "anti-**opiopathic drug**" are used interchangeably herein to refer to a composition of matter that induces a desired agonist, partial agonist (agonist/antagonist) or antagonist effect. The effect can be direct (e.g., administering an opiate) or indirect (e.g., administering an opioid peptide precursor or a vector for introducing a recombinant gene to modulate *in-situ* opioid or opioid receptor expression).

The term "ailment" encompasses human or animal conditions, disease states, disorders and syndromes, and is intended to avoid the unnecessary repetition of such alternatives as a group. Reference, for example, to an ailment specifically as a disorder or syndrome is intended to clarify the nature of that ailment.

**"Carriers"** or **"vehicles"** as used herein refer to pharmaceutically and/or veterinarily accepted excipients known to be suitable for use in drug formulation and administration. Carriers and vehicles useful herein include, but are not limited to any such material known in the art, which is nontoxic and does not interact with other components of the composition in a deleterious manner.

The terms **"endogenous"** and **"exogenous"** refer to the origin of a substance. An **"endogenous"** substance , e.g., insulin or the opioid peptide β-endorphin, originates from within the body. An **"exogenous"** substance, e.g., recombinant human insulin or the synthetic opiate oxycodone, originates from outside the body.

**"Extended**-"or **"sustained-release"** is defined for purposes of the present invention as the rate at which a drug must be released after administration in order to maintain a therapeutically effective blood (plasma) level for a minimum of about 6 hours, but preferably lasting 12 to 36 hours or longer. This term is also employed to identify a formulation having such a release profile.

**"Immediate-release"** is defined for purposes of the present invention as the rate at which a drug must be released after administration in order to maintain a therapeutically effective blood (plasma) level for period of about 6 hours or less. This term is also employed to identify a formulation having such a release profile.

A **"ligand"** is a molecule (such as an antibody, hormone or drug) that binds or otherwise attaches to another molecule (such as a receptor); such binding or other attachment is typically highly specific.

**"Nociceptive pain"** refers to the perception of discomfort from the application of an extrinsic noxious stimulus to the body (such as a burn or laceration). **"Pathologic** pain" refers the perception of discomfort and abnormal sensitivity arising out of an intrinsic insult (such as a tumor or tooth decay) or to the ongoing discomfort and abnormal sensitivity in previously injured tissue. **"Opiopathic** pain" refers to the perception of physical discomfort resulting from an abnormal concentration of one or more of the endogenous opioids, or involving the intrinsic blockade, underexpression or overexpression of one or more of the opioid receptors.

The term **"opioid"** refers broadly to all compositions of matter (endogenous or exogenous) that are chemically or structurally related to opium. **"Endogenous** opioids" or **"endogenous opioid peptides"** are the naturally occurring ligands for opioid receptors. **"Opiates"** are exogenous active agents that are chemically or structurally derived from opium and can bind to an opioid receptor, for example, including the natural products morphine, codeine and thebaine, and many semi-synthetic and synthetic derivatives; small molecules and peptides are within the scope of the term opiates (see, e.g., Goodman & Gilman Online, 11th Ed., Ch. 21 "Terminology").

The term **"Opioid-responsive Polyneuropathy Syndrome"** or **"ORPS"** has been coined for purposes of the present invention to identify an ailment that **involves** respiratory dysfunction (e.g., lingual, pharyngeal and/or laryngeal paresis/paralysis) plus one or more other forms of paresis/paralysis such as: neurogenic urinary bladder sphincter, lumbar and lumbo-sacral spine, and pelvis and pelvic limb paresis/paralysis.

The term **"opiopathy"** has been coined for purposes of the present invention to mean a neuropathic, polyneuropathic, neurologic or neurogenic ailment characterized by paresis/paralysis and involving an abnormal concentration of one or more of the endogenous opioids, or involving the blockade, underexpression or overexpression of one or more of the opioid receptors. The root term "opiopath" is employed in conjunction with word stems such as "ies" to give the plural and "ic" to give an adjective. Opiopathy encompasses **"hypo-opiopathy,"** i.e., a deficit of one or more of the endogenous opioids. Opiopathy also encompasses **"hyper-opiopathy,"** i.e., an excess of one or more of the endogenous opioids.

The term **"optional"** or **"optionally"** means that the subsequently described event or circumstance may or may not occur, and that the description includes instances where said event or circumstance occurs and instances in which it does not.

The term **"paresis/paralysis"** is defined for purposes of the present invention as partial or total loss of function in innervated muscle tissue.

The term **"pseudo-atrophy"** has been coined for purposes of the present invention to mean a decrease in muscle function and tone presenting as weakness and the visual appearance of wasting, which can be readily reversed (approaching normal function and appearance) by treatment with an anti-opiopathic agent.

The terms **"subject," "individual"** or **"patient"** are used interchangeably herein, referring to a vertebrate, preferably a mammal. The mammal is either a human or a non-human. Non-human mammals include but are not limited to, mice, rats, simians, farm animals, sport animals, and pets such as dogs and cats.

By **"therapeutically effective"** is meant a nontoxic amount of a drug, active agent or formulation in a quantity sufficient to provide a desired effect, e.g., treatment of an opiopathy.

The terms **"treat",** "treating" and "treatment"' as used herein means providing medical assistance to a mammal suffering from an ailment, and includes:
- prevention (i.e., causing clinical symptoms of the ailment not to develop),
- inhibition (i.e., arresting the development, severity and/or frequency of clinical symptoms of the ailment),
- regression (i.e., causing a reversal of clinical symptoms of the ailment, their severity and/or frequency),
- amelioration (i.e., to facilitate the healing of damage caused by the ailment) and/or
- cure (i.e., causing elimination of the cause and clinical symptoms of the ailment, for example, by facilitating a mammal's ability to produce a previously underexpressed opioid).
The present methods of treating opiopathy thus encompass treating predisposed individuals and clinically symptomatic individuals.

The term **"Upper Respiratory Obstructive Syndrome"** or **"UROS"** has been coined for purposes of the present invention to identify an ailment that involves at least two of: lingual, pharyngeal and/or laryngeal paresis/paralysis.

### ORIGINS OF OPIOPATHY

Long before advances in technology started making it possible to diagnose ailments at the genetic level, a physician would use the senses of sight, smell, hearing, touch (and even taste), intuition and deductive reasoning to make a diagnosis and prescribe a treatment. These experiences would then be applied to other cases with similar signs and symptoms. Extraordinary concurrences of such diagnoses and successful treatments would be communicated to colleagues and eventually become so well known as to be considered the "standard of care." The present invention arose in like fashion. Several years ago the present inventor observed an opiate-mediated reversal of paralysis of the arytenoids in a dog suffering from a critical respiratory obstruction; such paralysis was at the time believed attributable to the recurrent laryngeal nerve. About one week later the same dog appeared to have regained function of the urinary bladder sphincter (after ten years of total dysfunction) and after a second week the same dog regained mobility (after several years of near total hind leg paralysis). (See, Example 1.) Another respiratory emergency presented during the same period and was successfully treated using the same opioid. (See, Example 2.) In the years that followed, more than 35 cases with very similar neuropathic signs and symptoms have been successfully treated and documented in detail. The reproducible efficacy of opioids in treating these ailments has given rise to the present proposal of a common underlying cause and classification (i.e., opiopathy). Moreover, it appears that the muscles affected in these disorders are innervated by one or several of the paired cranial nerves that originate from the brain and are part of the peripheral nervous system. These cranial nerves in the canine are also found in humans and in other mammals, which also share a great deal of similarity in the structures innervated, and in the neuropathic signs and symptoms observed across sufferers of opiopathy.

Most knowledge about the endogenous opioids and their receptors pertains to the modulation of nociception (the perception of pain having its origin in an intrinsic or extrinsic insult or injury to previously healthy tissue), cough suppression, treatment of diarrhea and sedation without loss of consciousness. Endogenous opioid peptides are also known to be involved, for example, in the neuro-immune system (e.g., controlling IL-12 levels, which in turn plays a role in controlling migrating T-cells), in the neuro-psychiatric system (e.g., regulating mood modulation), in the neuro-muscular system (e.g., the production of acetylcholine, a neurotransmitter required at myoneural junctions for normal muscle contraction) and in the neuro-endocrine system (e.g., the production of thyroid stimulating hormone releasing factor in the hypothalamus, the production of adreno corticotropic stimulating hormone in the pituitary, and glucose metabolism). The present invention focuses on lesser-known roles of endogenous opioids and their receptors, and is premised on abnormalities in the levels of endogenous opioids and/or their receptors corresponding to a variety of ailments that were previously believed attributable to other or to unknown causes. These ailments have for the present purposes been defined as opiopathies. Additionally, the present invention is premised on the administration of exogenous opioids being effective to treat such opiopathic ailments, including opiopathic pain.

The observations and experimental treatments underlying the present invention arose in the present inventor's practice of veterinary medicine where dogs (predominantly aging dogs) had been observed over the years presenting with inappropriate panting and progressive difficulty in breathing. These signs and symptoms initially appeared consistent with the disorder Recurrent Laryngeal Nerve Paralysis "RLNP". At the time, the "gold" standard for diagnosing RLNP involved anesthetizing the patient with an ultra-short anesthetic (such as propothol or ketamine/valium, which would inhibit lingual and pharyngeal function to facilitate examination), opening the mouth, manually withdrawing the tongue and depressing the epiglottis to observe the movement and function of the arytenoids. The use of such anesthetics, withdrawal of the tongue and depressing the epiglottis unfortunately precluded observation of lingual and pharyngeal function. In the present inventor's practice, however, visualization of the arytenoids was accomplished without the use of potentially paralyzing anesthesia, and surprisingly did not give rise to choking, gagging and resistance to lingual withdrawal, as if the subjects had been anesthetized. In follow-up examinations, after treatment by administration of a therapeutically effective amount of an anti-opiopathic active agent, the choking, gagging and resistance to lingual withdrawal responses had returned in these un-anesthetized subjects, highlighting the absence of such responses during the prior examinations. As a result it was observed that dysfunction of any, and frequently two or all of the tongue, the pharynx and the larynx were involved in what had been called RLNP. The term upper respiratory obstructive system (or UROS) has been coined to describe the frequent involvement of multiple neuromyopathies in this ailment; where only a single neuromyopathy is involved the ailment is best identified as a specific paresis/paralysis (e.g., LiP). It has further been observed in the canine that the respiratory obstruction in UROS is attributable to LaP, LiP and/or PhP, as opposed to some other muscular or nervous function (e.g., the diaphragm). Dogs are known mouth breathers, as this also plays a role in heat exchange and regulation of their core body temperature. They are, however, capable of nasal respiration. It has surprisingly been observed that when a dog suffering from UROS is constrained to breath nasally (air passing directly into the trachea instead of passing the tongue, soft palate, arytenoids and vocal folds) such subject does not suffer from difficulty in breathing. While constrained nasal respiration is not a suitable long-term alternative for canine UROS sufferers (e.g., due to the other functions served by open-mouthed breathing) this observation does support a conclusion that the respiratory obstruction in UROS is attributable to LaP, LiP and/or PhP as opposed to some other muscular or neuropathic inadequacy, e.g., pulmonary dysfunction or diaphragmatic paresis/paralysis.

A significant number of these subjects presented with additional signs or symptoms including difficulties: in swallowing, standing (from a sitting position), in sitting (from a standing position), in walking, with urinary incontinence and/or with fecal incontinence, many suffering from two or more of these ailments simultaneously. Prior reports had described RLNP as one manifestation of a generalized neuromuscular syndrome also affecting peripheral strength, which was called "Laryngeal Paralysis - Polyneuropathy Complex". As with UROS, it was observed that both LLSP and PPLP (and even NUBSP) frequently co-presented with any or all of the respiratory ailments LaP, LiP and PhP (not just LaP). Heretofore, each of these ailments had typically required an independent therapeutic approach, whether surgical (e.g., removal of one or both vocal folds, arytenoid cartilages and tieback procedures) or pharmaceutical (e.g., corticosteroids to decrease laryngeal inflammation and thyroid supplementation). No single therapeutic approach had, however, been previously found to treat multiple aspects of such a syndrome other than palliatively. When palliative care stopped allowing such dogs to live a comfortable life with dignity, their owners often decided that their dogs should be humanely euthanized, especially in the cases of UROS, NUBSP, LLSP and PPLP. Even as recently as November 2005 it was reported that "[t]here is no proven efficacious drug therapy for similar polyneuropathies in humans." Griffin and Krahwinkel, "Laryngeal Paralysis: Pathophysiology, Diagnosis, and Surgical Repair," Compendium on Continuing Education for the Practicing Veterinarian, Nov. 2005, 857-869 at 862. It has surprisingly been demonstrated that the administration of an anti-opiopathic active agent (e.g., an opiate) can contemporaneously treat such diverse ailments, as described in greater detail below. The term opioid-responsive polyneuropathy syndrome (or ORPS) has been coined in order to clarify that sufferers of this syndrome can be experiencing respiratory difficulty for reasons other than or in addition to laryngeal paresis/paralysis, in view of the diversity of ailments encompassed by the syndrome, to highlight the opiopathic nature of the ailment and its amenability to treatment by administering an anti-opiopathic active agent. The adoption of such terminology is consistent with current medical practice where, for example, in cases where the sign(s) and/or symptom(s) of an ailment are readily observable and correlate with a particular effective treatment to the extent that its treatability can describe the ailment (such as in the case of thyroid-responsive dermatosis, where irrespective of measurable hypothyroidism the administration of thyroid hormone provides effective treatment for sufferers exhibiting the corresponding signs and symptoms).

Following several initial experiences where the administration of a sustained release opioid (OxyContin®) had provided effective treatment for what would now be diagnosed as OPS involving UROS concurrently with LLSP, PPLP and NUBSP (see Examples 1 and 2), it became increasingly apparent that a significant number of veterinary subjects suffered from a combination of such symptoms. Additional anesthetic-free examinations of such subjects provided evidence of paresis/paralysis in the muscles corresponding to the symptoms (i.e., lingual, pharyngeal, laryngeal, esophageal, urinary bladder sphincter, lumbar- and lumbo-sacral spine, and pelvis and pelvic limb). In two cases where upper respiratory difficulty had been surgically treated (tieback surgery) the subjects were observed as having continued difficulty in swallowing, notwithstanding the physical constraints holding their apparently obstructing tissues open. Anesthetic-free oral examination revealed that the swallowing difficulty was being caused by a loss of function in other airway structures. These subjects also suffered from difficulty in walking (with apparent atrophy to the musculature of the lumbar and lumbo-sacral spine, the pelvis and pelvic limbs) together with urinary incontinence. OxyContin was prescribed. Upon examination following one or two days of OxyContin treatment, it was observed that those airway structures that were not tied back had resumed normal functioning. It was also observed that these subjects' difficulty with urinary incontinence had improved, difficulty with walking had improved and that the corresponding muscles, previously having appeared to have atrophied, were returning to normal appearance (in terms of size, tone and strength).

It is accepted that ailments involving paresis/paralysis result in muscular atrophy (loss of muscle size, tone and function), the prognosis for which has typically been a limited return to function and appearance following an extended period of "successful" rehabilitation. For example, a fractured femur or tibia requires immobilization in a cast for a period of 4 to 12 weeks. Upon removal of the cast, the limb's musculature will appear shrunken and flabby, and will have experienced a significant loss of strength (i.e., atrophy or more particularly for purposes of the present invention, "disuse atrophy"). Only after months of physical therapy and conscientious exercise will the limb return to normal appearance and function.

Subjects in the underlying studies had suffered paresis/paralysis for periods of months and even years prior to treatment in accordance with the present invention. Upon examination, their affected musculature appeared shrunken, flabby and incapable of function (even to the extent of demonstrating no gag reflex when touching the back of the throat, for example, with a tongue depressor). Treatment of UROS and OPS patients in accordance with the teachings of the present invention has provided reproducible evidence of a return to normal appearance and function in the muscle tissues of the tongue, pharynx and larynx within two to six hours after receiving an effective dose of an anti-opiopathic drug (e.g., an opioid). Once an effective dose has been established, neurogenic urinary bladder sphincter function has typically resolved after about seven days. Muscles of the lumbar and lumbo-sacral spine, pelvis and pelvic limbs have typically regained normal muscular appearance and function after about two weeks. Such dramatic recoveries were previously unheard of in cases of muscular atrophy. Significantly atrophied muscle simply does not return to seemingly normal function and appearance over a period of weeks, much less over a few hours. It has, therefore, been concluded that the loss of muscle function and appearance cannot be properly diagnosed as atrophy and must be the result of a distinctly different physiologic process. Such loss of muscle function and appearance, which proves treatable over relatively short periods of time by administration of an anti-opiopathic drug, has therefore been identified as a distinct ailment for which the term "pseudo-atrophy" has been coined.

Without wishing to be confined to any specific theory as to why opiate active agents produce such dramatic results in treating paresis/paralysis, it is submitted that abnormalities in the levels of endogenous opioids and/or their receptors correspond to a variety of ailments that were previously believed attributable to other or to unknown causes. The pathology leading to pseudo-atrophy and the paresis/paralysis observed in opiopathies appears attributable to the nervous system, not to the muscles themselves. These muscles remain able to function; they are simply not being provided with the stimuli required to do so, which is believed attributable to abnormal levels of endogenous opioids and/or their receptors.

Still another aspect of opiopathy relates to pain. Pain plays an integral role as part of the body's normal defense mechanism, warning of contact with potentially damaging environmental insults and initiating behavioral and reflex avoidance strategies. As discussed above, the endogenous' opioids modulate the perception of pain by being released in response to the application of a noxious stimulus to the body (i.e., nociceptive pain). Endogeonous opioids function by inhibiting transmission of the neurologic signals indicating pain (e.g., by inhibiting release of acetylcholine, dopamine and norepinephrine, or by hyperpolarizing their target neurons to reduce firing rates). For example, a cut or a bum will be perceived as painful particularly close to the time of the injury, but the perception of such pain will tend to dissipate long before the wound has completely heated; the perception of pain from the wound diminishes due to up-regulated production of endogenous opioid peptides. The administration of exogenous opioids similarly act to decrease the perception of pain (whether Nociceptive or Pathologic) for the period of time over which the drug is effective.

Opiopathic pain differs from nociceptive or pathologic pain in that it can arise without application of a noxious stimulus (e.g., in conjunction with an opiopathic ailment). Opiopathic pain, while perceived as a physical sensation, is not the result of an inflicted wound or intrinsic injury; it is a sensation perceived from a part of the body where opioid levels are abnormal and therefore fail to inhibit the transmission of neurologic signals indicating pain even though there is no injury. The resulting pain can be treated by providing an amount of the correct anti-opiopathic drug effective to re-establish a normal opioid balance.

One familiar example of nociceptive pain would be suffering from a broken leg; when the right opiate is administered to a subject having a broken leg, the leg doesn't hurt as much, but, it is still broken and incapable of use. An example of opiopathic pain can be found in multiple sclerosis, where the inability to voluntarily control a muscle or muscle group is accompanied by the perception of sometimes debilitating pain. When the right opiate is administered to an MS sufferer, the perception of pain from afflicted nerves diminishes; unlike nociceptive pain, however, the muscles innervated by those nerves regain their function and appearance. Thus, in addition to reporting the category identified as opiopathic pain, the present description also provides active agents, formulations and methods for the treatment thereof.

Endocrine manipulation has been show to effect the hypothalamic and pituitary contents of met-enkephalin and beta-endorphin, suggesting additional sites having opioid modulation and the potential for opiopathy. In the pituitary, gonadectomy decreases beta-endorphin content in both the anterior lobe and neuro-intermediate lobe. Orchidectomy results in a decrease while ovariectomy leads to an increase in anterior lobe met-enkephalin contents. Adrenalectomy leads to an increase in beta-endorphin contents only in the anterior pituitary lobe. Hypothyroidism induced by propylthiouracil treatment is accompanied by a decrease of beta-endorphin in the neuro-intermediate lobe and a decrease in met-enkephalin in the anterior lobe while thyroidectomy entails a decrease in met-enkephalin in the anterior lobe only. Chemically induced diabetes mellitus results in a decrease in beta-endorphin content in the hypothalamus and the neuro-intermediate lobe, and a reduction in met-enkephalin level in the anterior and neuro-intermediate lobes. (Paraphrased from, Tang, F., "Endocrine control of hypothalamic and pituitary met-enkephalin and beta-endorphin contents." Neuroendocrinology, 1991;53 Suppl. 1:68-76.)

Irrespective of the present proposals regarding mechanism of action, nomenclature and the like, the fact remains that a group of ailments involving paresis/paralysis, which had previously been considered untreatable, have been reproducibly treated by administering a therapeutically effective amount of an opiate.

### AILMENTS

The ailments treated in accordance with the present description are typically neuropathic, polyneuropathic, neurologic or neurogenic, particularly those characterized by paresis/paralysis, and can now also be referred to as opiopathies. Additionally included among the treatable ailments are opiopathic pain and pseudo-atrophy. Still other groups of opiopathic ailments include those pertaining to immune surveillance (e.g., opioid-related autoimmune diseases such as thrombocytopenia), tumor surveillance, and neuroendocrine modulation. While most of the ailments treated to date have involved "hypo-opiopathy," treatment of the inverse condition "hyper-opiopathy" (for example, the excess of an endogenous opioid interfering with signal transmission and giving rise to numbness or lack of tactile sensation) is also contemplated. Thus, any such ailment arising as a function of the abnormal concentration of one or more of the endogenous opioids, or involving the blockade, underexpression or overexpression of one or more of the opioid receptors is within the scope of opiopathic ailments.

Endogenous opioid concentration has been identified as a common factor in widely diverse ailments. As discussed below, opiopathies can be described with great specificity and are proposed to correspond with a variety of the presently identified disease states. The present teachings can also facilitate taking a broader approach, for example in viewing health problems in the aging population. Prevalent among the residents of senior assisted care facilities are difficulties in swallowing (dysphasia) and shuffling of the feet while walking; these are commonly and very simply just chalked up and surrendered to as the signs of aging. Many such senior citizens also suffer from incontinence and leak urine. Corresponding signs and symptoms have now been documented in the canine and unexpectedly shown responsive to treatment with opiates. These results are sufficiently compelling to warrant the consideration of opiopathy in the diagnosis and opiates in the treatment of such "neuropathic" ailments in humans and other mammals.

One group of treated ailments includes (without limitation) lingual, pharyngeal, laryngeal, esophageal, urinary bladder sphincter, lumbar and lumbo-sacral spine, and pelvis and pelvic limb paresis/paralysis, whether identified alone or as part of a larger neurologic or neurogenic syndrome such as Opioid-responsive Polyneuropathy Syndrome or Upper Respiratory Obstructive Syndrome. Also included are snoring and the swallowing disorders characterized by paresis/paralysis. With regard to ailments involving paresis/paralysis of the urinary bladder sphincter it should be noted that the resulting urinary incontinence differs from incontinence associated with stress, urge or overload. Neurogenic urinary bladder sphincter incontinence, involves passive, persistent leakage of urine resulting from neuropathic sphincter dysfunction.

Examples of treated ailments seen individually or as part of a larger neurologic or neurogenic disorder or syndrome, include, but are not limited to any single ailment or combination of the following ailments: cardiomyopathy, centrally mediated depression, congestive heart failure, and paralytic intestinal ileus.

Examples of polyneuropathic syndromes treatable in accordance with the present description include, but are not limited to any single ailment or combination of the following: Multiple Autonomic Nervous System Dysfunction, Multiple Sclerosis, Myasthenia Gravis, Parkinson's Disease, Post-Polio Syndrome and ALS. Other ailments having similar signs and symptoms, such as Addison's Disease, Muscular Dystrophy, Fibromyalgia, Spinal Muscle Atrophy, Spinal Muscle Atrophy with Respiratory Distress Type 1, and Sarcopenia can be readily tested in accordance with the present teachings to confirm such ailments' classification as opiopathies and their responsiveness to the present methods of treatment. Similarly, opiopathic involvement and treatments in immune surveillance (e.g., opioid-related autoimmune diseases such as thrombocytopenia), tumor surveillance (α-melanocyte-stimulating hormone ("α-MSH")), neuroendocrine modulation, and "hyper-opiopathy" (for example, the excess of an endogenous opioid interfering with signal transmission and giving rise to numbness or lack of tactile sensation) are also envisioned, particularly as the roles of endogenous opioids involved in the various aspects of these ailments are further elucidated.

Almost every muscle and group of muscles in the body has as its pair an opposing muscle or group of muscles, for example, the biceps and triceps, If a single opioid was responsible for the contraction of both muscles in such a pair, they would contract at the same time and make movement impossible. Thus, different opioids (e.g., an agonist and antagonist) can be envisioned as controlling a given muscle pair. MS, Lou Gherig's Disease and Parkinson's Disease are currently being treated with limited success using the opiate antagonist naltrexone. Consistent with the foregoing teachings , the treatment of such ailments would likely entail administration of more than a single anti-opiopathic agent (e.g., both an opiate antagonist and an opiate agonist) to re-establish neuromuscular transmitter reserves necessary to communicate the instructions of the brain, for example to contract the biceps while relaxing the triceps.

By way of example, and without wishing to be bound by any particular theory or mechanism by which the therapeutic methods of the description function, it is believed that Multiple Sclerosis is not (as commonly discussed) primarily attributable to demyelination and consequential neuronal inefficiency, but, is instead a hypo-opiopathic condition impacting neuronal transmission in at least two concurrent but distinct manners. Demyelination has been associated with a particular component of the immune system, the T-effector cell, which is capable of crossing the blood-brain barrier and known to erroneously attack myelin as "not self." The modulation of T-effector cells has recently been associated as corresponding to levels of Interleukin-12 (IL-12), which in turn modulates the T-regulatory cells that prevent T-effector cells from roaming freely into the CNS. It has been independently demonstrated that IL-12 concentration is modulated by certain opioids. Another of the roles identified for such opioids is as neuronal and neuromuscular junction transmitters. Thus, a decrease in certain endogenous opioid levels would simultaneously diminish neuronal and neuromuscular junction transmission and up-regulate IL-12/T-effector cells leading to the demyelination also seen in the disease. In that regard, it is submitted that the amount of demyelination found in MS sufferers could not realistically be expected to interfere with neuromuscular transmission to the extent seen in MS suffers. Still further support stems from observation of an individual recently diagnosed as having MS who suffered episodes of what is commonly termed "emotional incontinence," from which treatment was obtained by administering vicodin, as described in greater detail in Example 10 below. It is further submitted that the foregoing may prove to be a kappa opioid modulated process, particularly given certain recent publications indicating a kappa opioid receptor affinity of oxycodone. Specific mechanisms of action notwithstanding, it is now possible to provide effective treatment for MS by administration of an opiate.

Many sufferers of ailments (such as Post-Polio Syndrome, ALS and MS) experience considerable pain erroneously classified for example as nociceptive pain, headache or peripheral pain, for which traditional pain-relieving strategies have been used (e.g., aspirin, ibuprofen, acetaminophen and low doses of narcotic-containing mixtures such as vicodin and Tylenol/codeine, albeit at dosage levels now understood to be insufficient for treating opiopathies). In one reported study (Kalman et al., European Journal of Pain (2002) 6:69-80) a group of MS patients experiencing central pain ("CP," which for purposes of the study included trigeminal neuralgia) were treated intravenously with placebo and with 1.0 mg/ml morphine in physiological saline, to evaluate the desirability of using stronger analgesics for the treatment of such pain. The results were reported as showing that neuropathic pain is poorly responsive, but not totally unresponsive to opioids. To the extent shown, opioid responsiveness only occurred after high doses of morphine, and were concluded not to support the routine use of strong opioids in MS patients with CP. The foregoing report is distinguishable when viewing MS as an opiopathy in which CP is an example of opiopathic pain. Consistent with the teachings of the present description, alternative anti-opiopathic active agents and/or morphine dose escalation should be evaluated in such patients. Such generally ineffective use of aspirin, ibuprofen, acetaminophen and low doses of vicodin or Tylenol/codeine is believed due to the prior lack of understanding about opiopathies and opiopathic pain,

Those skilled in the art will appreciate that opiopathies may result from a variety of causes and can likewise be treated employing a variety of approaches. For example, opioids are involved in the manufacture of Thyroid Stimulating Hormone Releasing Factor ("TSHRF") in the hypothalamus, which in turn acts on the pituitary gland to make Thyroid Stimulating Hormone ("TSH"), which in turn acts on the thyroid to make Thyroid Hormone ("TH"). All but two of the dogs involved in the research leading to the present invention were diagnosed hypothyroid (using free T4 by E.D. and TSH). While the administration of replacement thyroid hormone was effective to return T4 levels to normal, it had no effect on the paresis/paralysis observed in these subjects. Surprisingly, administration of an anti-opiopathic active agent (e.g., OxyContin) had the effect of treating their paresis/paralysis and returning the subjects' T4 levels to normal. Thus, hypothyroidism can be treated through administration of an anti-opiopathic agent, particularly where the ailment is hypo-opiopathic in nature and exists concurrently with an opiopathic paresis/paralysis. Hypothyroidism caused by iodine deficiency, bilateral tumor infiltration, thyroidectome or the like, is not expected to respond to treatment with an anti-opiopathic agent.

Identification of the specific nerve or group of nerves associated with a neurologic or neurogenic disorder or polyneuropathic syndrome with their attendant clinical signs and symptoms, and documenting which of the anti-opiopathic active agents used in the present description effectively treats the associated disease signs and symptoms, further provides a unique opportunity to apply this knowledge to the treatment of other disease states caused by a neuropathic disorder or polyneuropathic syndrome, which contain some or all of these same effectively treated clinical signs and symptoms as part of their signalment.

### DIAGNOSIS AND TESTING

The diagnosis of opiopathies can be accomplished through art-recognized clinical evaluation of a presenting subject's condition as corresponding to one or more of the above-mentioned ailments. For example, the clinical diagnosis of UROS initially entails observation of breathing difficulty. In veterinary medicine, particularly for a dog, a simple tentative diagnosis can be performed by holding the subject's mouth closed. If the subject is able to breath without obstruction when the mouth is held closed, and difficulty in breathing (e.g., gasping, panting, choking, gagging) returns when the mouth is permitted to reopen, a positive diagnosis of opiopathy can be made. Such diagnosis can be further verified by doing a simple visual examination (by quantitation of various symptoms, in the discretion of the treating veterinarian). If UROS is tentatively diagnosed, administration of a known effective anti-opiopathic agent will confirm the diagnosis if the UROS symptoms resolved.

Alternatively in accordance with the present description, a presenting subject can be evaluated using diagnostic technology such as ELISA, radio receptor assay (RRA) and like assays for measuring endogenous opioid, precursor and/or metabolite concentrations in laboratory specimens (e.g., from blood, serum, plasma, urine, synovial fluid, cerebral/spinal fluid, lymphatic fluid or from a tissue biopsy) or *in situ,* e.g., by positron emission tomography (PET) and the like.

The present description further encompasses a method for testing or identifying an anti-opiopathic active agent, for example an opioid compound or a pharmaceutical formulation, for effectiveness in treating a neurologic or neurogenic disorder or syndrome, including the steps: (a) evaluating the function of a muscle group or an organ of a subject, wherein the subject suffers from a neurologic or neurogenic disorder or syndrome, (b) administering an opioid compound or pharmaceutical formulation to the subject, (c) re-evaluating the muscle or organ's function, and (d) determining whether said opioid compound or pharmaceutical formulation provided effective treatment. The method involve repeating steps (a)-(d) for testing or identifying more than one opioid compound or pharmaceutical formulation. When necessary, the method can further involve repeating steps (b) and (c) one or more times for each opioid compound or pharmaceutical formulation tested. The function evaluated can be lingual, pharyngeal, laryngeal, esophageal, urinary bladder sphincter, lumbar and lumbo-sacral spine, or pelvis and pelvic limb control or function. Preferably, the step of evaluating the function of an organ involves grading the function using a grading system. This testing method can be employed to evaluate the potential effectiveness of presently known opioid active agents, to characterize a known therapeutic agent previously unassociated with anti-opiopathic activity, or to identify the utility of a novel therapeutic agent.

In order to clinically diagnose and/or evaluate the effectiveness of a dose of an anti-opiopathic active agent or formulation, over time, when being used to treat an opiopathic ailment, the following grading protocol can be employed to quantify and document function of the target muscle(s) and/or organ(s). Evaluations are conducted at the start, optionally at midpoint(s) and at the conclusion of treatment regimen, typically spanning from two to eight weeks.

In grading the evaluations, an organ is considered to be neurologically normal when there is no neuropathology affecting its function. Because in most cases an organ can be clearly identified as having normal neurologic function, decreased neurologic function (paresis), or no neurologic function (paralysis), the documenting of the degree of remaining neurologic function of an organ lends itself to a simple grading system. An organ that functions normally and has no observable signs or symptoms is considered to be neurologically normal and receives the lowest score (0). An organ that has no function as a result of a neurologic/neurogenic ailment and is paralyzed, receives the highest score (10). An organ that has a partial loss of function (paresis) receives a score between (1) and (9), depending on the degree of function remaining. A decrease of the score of a function after administration of an anti-opiopathic active agent or formulation, indicates effectiveness roughly proportional to the relative decrease. Application of this grading system to the above-described ailments involving paresis/paralysis proceeds as follows:
- Lingual: Grade (0) = normal lingual musculature, movement while swallowing, and withdrawal in response to pinching with a hemostat. Grade (10) = pseudo-atrophy of lingual musculature, inability to swallow a bolus of food, audible choking and gagging, and no lingual withdrawal in response to pinching with a hemostat.
- Pharyngeal: Grade (0) = normal swallowing of a bolus of food or liquid, no audible obstructive airway sounds, and no audible choking or gagging sounds. Grade (10) = no ability to swallow a bolus of food of liquid; audible obstructive airway sounds with choking and/or gagging.
- Laryngeal: Grade (0) = normal unobstructed flow of air into and out of the larynx with normal vocalization. Grade (10) = obstructed flow of air into the larynx with obstructed upper airway sounds, choking and gagging noted, and loss of vocalization. An exemplar of a grading system for laryngeal function, further divided into breathing, swallowing, laryngiospasm, jaw tone, and overall exposure of the larynx for examination, is described in Gross et al. (J. Am. Animal Hosp. Assoc. 38:503-6 2002).
- Esophageal: Grade (0) = normal passage of a bolus of food or fluid, after swallowing, from the back of the throat into the stomach. Grade (10) = severely delayed or impaired passage of a bolus of food or fluid, after swallowing, from the back of the throat into the stomach, due to a lack of peristaltic contractions within the esophagus, with possible secondary symptoms of regurgitation, esophageal pain and halitosis.

- Urinary Bladder Sphincter: Grade (0) = normal urinary bladder sphincter function, normal ability to store and pass urine. Grade (10) = no urinary bladder sphincter function with continual leaking of urine out of the bladder and subsequently out of the urethra, and secondary consequences including urine scald, moist dermatitis, urethritis, cystitis, nephritis.
- Lumbar and Lumbo-Sacral Spine: Grade (0) = normal tone and function of muscles that are responsible for moving the lumbar and lumbo-sacral spine while bending, moving the back, and supporting the lower torso. Grade (10) = apparent wasting and loss of all tone of the muscles that are responsible for movement of the lumbar and lumbo-sacral spine rendering the body incapable of supporting the back and lower torso and thus preventing any voluntary movement of this part of the body.
- Pelvis and Pelvic Limb: Grade (0) = normal tone and function of muscles that are responsible for extending and flexing the joints of the pelvis and pelvic limbs. Grade (10) = apparent wasting and loss of all tone of the muscles that are responsible for extending and flexing the joints of the pelvis and pelvic limbs, rendering them incapable of supporting the body and precluding ambulation.

Diagnosis of pseudo-atrophy can be accomplished by various functional/clinical examination approaches. One approach for clinical identification of pseudo-atrophy is by correlating a loss of muscle tone without loss of mass as corresponding to pseudo-atrophy. Finally, a functional approach involves administering an anti-opiopathic active agent to a subject suspected of suffering from pseudo-atrophy and observing for return of muscle function and tone over a relatively short period of time (e.g., over a matter of days/weeks as opposed to months/years, and absent intensive physiotherapy).

In the laboratory analysis aspect , a sample (e.g., blood, plasma, urine, cerebral/spinal fluid, or a tissue biopsy) is obtained from presenting subject. Preparation of the specimen for analysis can be accomplished utilizing art-recognized techniques appropriate to the testing methodology and equipment. The endogenous opioid content of the specimen is determined (e.g., by competitive binding to an ELISA agent specific for an endogenous opioid known to be associated with the subject's suspected condition, or with a panel of such agents such as in cases where there exists no specific association between the condition and an opioid). The ELISA agent can be an endogenous opioid receptor, for example, bound to a substrate. The measurement of opioid peptide plasma levels can be determined by radio receptor assay (RRA), for example as described by Odou, et al. (Nephrol. Dial. Transplant (2001) 16:1953-1954). Alternatively, the specimen's opioid content can be directly analyzed employing UV or IR spectroscopy, HPLC mass spectroscopy and the like.

In the *in situ* analysis aspect, an opioligand labeled with a physiologically acceptable radionuclide (e.g., ¹¹C, ¹³N, ¹⁵O or ¹⁸F) is administered to a presenting subject, followed by PET to measure as a function of time of the distribution of that nuclide in a structure of interest (see, e.g., WO/2005/094686 A2). The ligand can be an endogenous or exogenous opioid. The ligand can be an opioid active agent specific for an opioid receptor known to be associated with the subject's suspected condition, for example, ¹³N- or ¹⁵O-radiolabeled oxycodone. Procedurally, in one method according to the description a subject is first scanned without administration of any medications to establish a baseline, followed by administration of a cocktail containing differentially labeled exogenous opioids. After waiting a sufficient period, the scan is repeated and the distribution of bound opioids recorded.

Ultimately, the hardware employed to perform *in situ* diagnosis of opiopathies will employ one or more databases including baseline standard concentrations for the opioids and receptors that have been established as relating to the various opiopathies, and software for measuring and comparing a particular subject's endogenous concentrations and dispersions thereagainst, optionally correlating discrepancies between normal baseline and observed measurements with probable diagnoses. Such database building procedures can be verified by testing a subject known to suffer from an opiopathy, by PET scanning to identify an absence of endogenous opioid(s) from affected receptors. In like manner, such PET technology and databases can be employed to identify new opiopathic ailments and confirm the status of other previously identified ailments as opiopathies.

Similarly, the diagnosis of opiopathic pain can be accomplished by non-invasive, pathologic and functional approaches. The non-invasive approach can be carried out employing PET as described above. The laboratory analysis approach: entails obtaining a specimen from a subject suspected of suffering from opiopathic pain and measuring for opioid concentration. Finally, a functional approach involves administering an anti-opiopathic active agent to a subject suspected of suffering from opiopathic pain and observing for a lessening of such pain without the occurrence of opioid side effects associated with administration of opioids to patients having normal endogenous opioid levels.

Still another aspect of the diagnostics enabled as part of the present description pertains to the generation of an opiate responsiveness profile, to ascertain whether a particular anti-opiopathic active agent or any of a group of active agents is likely to be effective for a given subject. It is well recognized that certain individuals respond to some, but not to other opiates (for example, some people respond to morphine while others have rio response, but do respond well to Demerol, and vice versa). Without knowing which opiates work on a given patient, the physician (most notably anesthesiologists) is left to determine effectiveness by trial and error, sometimes at a point in treatment where time is critical. The above-described methodologies and devices can also be employed to generate such profiles, again based upon identifying a prevalence of endogenous opioids and the binding of labeled test opioids. By analogy, the methodology and devices can also be employed to detect excessive opioid levels, thereby diagnosing hyperopiopathic ailments and screening for potential side effects from synthetic opioids and overdose situations.

In a testing method for evaluating novel anti-opiopathic compounds according to the present description a novel compound is first synthesized to include a physiologically acceptable radionuclide (e.g., ¹¹C, ¹⁹N, ¹⁵O or ¹⁸F). The labeled test compound is administered to a subject known to have an opiopathic condition, followed by the measure as a function of time of the distribution of that nuclide in a structure known to be associated with the condition of interest. Probability of activity will be proportional to a compound's selectivity. Such information can be employed as key criteria in traditional rational drug design programs and in the computer modeling of novel drugs.

Similarly, identification of the receptors associated with particular opiopathic conditions (e.g., by PET testing of a panel of labeled receptor-specific opioids) can be employed to identify endogenous precursors, synthetic pathways and modulators that can be employed as therapeutic active agents in their own right or as targets for intervention. For example, a therapeutic down-regulating the cytochrome p54 clearance pathway in the liver could increase the effective half-life of oxycodone or a corresponding endogenous opioid the excessive clearance of which gives rise to a hypo-opiopathic condition.

It will also be understood that the present disclosure enables additional research methodology, for example to further elucidate causes of opiopathies and to evaluate additional therapeutic approaches. In that regard, another aspect of the description provides: methods of determining whether an endogenous substance (e.g., an enzyme, an opioid, an opioid precursor or an opioid receptor) is associated with an opiopathy; methods of determining whether a substance is an active anti-opiopathic agent; methods of determining whether an ailment is opiopathic. Still another aspect of the description provides reagents and kits for use in the foregoing methods.

One such approach can be premised on exogenous active anti-opiopathic agents and receptors with which they bind. In a corresponding method, an active anti-opiopathic agent is administered to a subject having an opiopathy or to a tissue or cell sample obtained from such a subject, followed by identification of the receptor(s) with which the agent binds, identification of the endogenous substance(s) that bind with such receptor(s) and any precursor(s) thereto, and determining whether such endogenous substance(s) is produced at normal or abnormal levels in such subject or sample.

Another such aspect employs an opiopathic recombinant animal (e.g., a mouse) that has been engineered not to express an endogenous opiopeptide or its precursor or to exhibit a particular opiopathy. Such recombinant animals can be engineered and reproduced using art-recognized methodology (for example, as described with respect to the *nmd* mouse generated by The Jackson Laboratory. See, Maddatu, et al., Human Molecular Genetics, Vol. 13, No. 11, 1105-1115). Upon selectively blocking the expression of one or more endogenous opiopeptides or precursors, the animal is examined for paresis/paralysis and/or opiopathic pain. The absence of paresis/paralysis or opiopathic pain indicates that the blocked opiopeptide or precursor is not associated with opiopathy. The presence of paresis/paralysis or opiopathic pain indicates that the blocked opiopeptide or precursor is associated with opiopathy, in which case correlation of paresis/paralysis or opiopathic pain with that exhibited in an opiopathic ailment indicates the involvement of such opiopeptide or precursor in the ailment. Confirmatory testing is conducted by administering an exogenous source of the missing opiopeptide, precursor or an equivalent opiate and observing the animal for whether the paresis/paralysis or opiopathic pain is treated. Test substances can also be administered to such animals, wherein treatment of paresis/paralysis or opiopathic pain corresponds to anti-opiopathic activity.

Still another confirmatory testing method involves blocking the activity of a test anti-opiopathic active agent by administering naloxone and naline followed by said agent and examining for a return of opiopathic symptoms. The naloxone and naline are then withdrawn and the agent re-administered; treatment of opiopathic symptoms confirms activity.

### ACTIVE AGENTS

An (anti-opiopathic) active agent useful in the practice of the present invention can be an opiate, an opioid peptide precursor or a vector for introducing a recombinant gene to promote in-situ opioid or opioid receptor expression. The active agent can be an agonist, a partial agonist (agonist/antagonist) or an antagonist, and can be selective for binding to only one or a selected mixture of the mu (µ), delta (δ), kappa (κ) and N/OFQ opioid receptors. Also contemplated within the scope of the invention is the administration of more than one anti-opiopathic agent, for the purpose of balancing the effect of the treatment or selectively modulating multiple targets. Such anti-opiopathic active agent(s) can optionally be co-administered with other compatible pharmaceutically active agents, for example, agents otherwise employed in treating a given ailment (e.g., an MS sufferer can receive sustained release oxycodone hydrochloride and interferon beta 1-a, interferon beta 1-b, glatiramer acetate, mitoxantrone or natilizumab).

It should be noted that a given opioid active agent can be effective for treating different indications in different parts of the body. By way of example, while oxycodone has been found effective for reversing paresis/paralysis in the larynx, this active agent is also effective for treating urinary bladder sphincter paresis/paralysis. It further appears that individual endogenous (or exogenous) opioids can perform distinctly different functions depending on the physiologic system and environment in which they are found or administered, particularly including a subject's endogenous opioid levels. In other words, administering morphine to a subject having normal endogenous opioid levels may cause euphoria, whereas the same dose of morphine may not cause euphoria in a subject suffering from a diminished level of the corresponding endogenous opioid.

An endogenous opioid associated with a subject's opiopathic condition can be identified by a diagnostic method of the present description, and an anti-opiopathic active agent specific for the identified endogenous opioid is administered in an amount sufficient to treat the opiopathy, e.g., by normalizing the endogenous opioid level. Commonality in receptor class and endogenous opioid parent can be employed as criteria for selecting among alternative anti-opiopathic active agents when developing a treatment regimen for a given subject. The diagnosis and treatment of some opiopathic conditions will entail the identification and administration of more than one endogenous opioid or its replacement.

Examples of opiates that can be used in the present invention include, but are not limited to: alfentanil, allylprodine, alphaprodine, anileridine, benzylmorphine, beta-hydroxy 3-methylfentanyl, bezitramide, buprenorphine, butorphanol, carfentanil, clonitazene, codeine, cyclazocine, desomorphine, dextromoramide, dezocine, diacetylmorphine (heroin), diampromide, dihydrocodeine, dihydroetorphine, dihydromorphine, dimenoxadol, dimepheptanol, dimethylthiambutene, dioxaphetylbutyrate, dipipanone, eptazocine, ethoheptazine, ethylmethylthiambutene, ethylmorphine, etonitazene, etorphine, fentanyl, hydrocodone, hydromorphone, hydroxypethidine, isomethadone, ketobemidone, LAAM, levallorphan, levorphanol, Ilevophenacylmorphan, lofentanil, meperidine, meptazinol, metazocine, methadone, O-methylnaltrexone, metopon, morphine, myrophine, nalbuphine, nalorphine, naloxone, naltrexone, narceine, nicomorphine, norlevorphanol, normethadone, normorphine, norpipanone, opium, oxycodone, oxymorphone, papaveretum, pentazocine, phenadoxone, phenomorphan, phenazocine, phenoperidine, piminodine, piritramide, propheptazine, promedol, properidine, propiram, propoxyphene, remifentanil, sufentanil, tildine, tramadol, and the pharmaceutically acceptable salts thereof.

In one embodiment, the anti-opiopathic active agent is one of the naturally occurring opiates codeine, morphine, noscapine, papaverine or thebaine. In another embodiment, the anti-opiopathic active agent is a semi-synthetic opiate, preferably hydrocodone, hydromorphone, levorphanol, metapon, nalorphine, naloxone, naltrexone, oxycodone, oxymorphone or tramadol. In another embodiment, the anti-opiopathic active agent is a synthetic opiate, preferably ethoheptazine, fentanyl, levorphanol and congeners, meperidine and congeners, methadone and congeners, phenazocine or propoxyphene. While drugs such as heroine and opium can be employed in the practice of the invention, they have been excluded from the listings of preferred opiates for reasons of practicality (e.g., acceptability) rather than efficacy. Alternatively, the active agent can be an endogenous opioid precursor or compound that modulates opioid synthesis *in situ.*

The anti-opiopathic active agent can be a phenanthrene, a phenytheptylamine or a phenylpiperidine. Examples of phenanthrenes include, but are not limited to: codeine (Tylenol 3, 4), etorpine (Immobilon), hydrocodone (Vicodin, Lorcet), hydromorphone (Dilaudid), morphine (MS Contin), oxycodone, and oxymorphone (Numorphan). Commercially available oxycodone formulation include, but are not limited to: Endocet, Oxycet, Oxycodan, OxyContin, Percocet, Percodan, Roxicet, Roxilox, Roxiprin, Roxycodone, Supeudol and Tylox; Remoxy, an abuse-resistant formulation, is being tested in clinical trials. Examples of phenylheptylamines include, but are not limited to: dimeheptanol (methadol), dimenoxadol, dipipanone, isomethadone, methadone, methadyl acetate, and propoxyphene (Darvon). Examples of phenylpiperidines include, but are not limited to: alfentanyl (Alfenta), alphaprodine, beta-promedol, carfentanyl, fentanyl (Sublimaze), lofentanil, meperidine (Demerol), properidine, and sufentanil (Sufenta).

Also contemplated is the use of a novel anti-opiopathic active agent identified through practice of a previously described testing method, e.g., in a method of treatment or testing, or a pharmaceutical or veterinary formulation of the invention.

As indicated above, different opiates can be combined for administration. In one embodiment, a first component is an opioid agonist and a second component is an opioid antagonist. Preferably, the second component blocks at least a portion of the action of the first component. This blocking results in a reduction of adverse side-effects, such as one or more of addiction, constipation, and sedation. In a preferred combination, the first component is hydrocodone, morphine, oxycodone or tramadol, and the second component is naltrexone (most preferably an ultra-low concentration of naltrexone). The first and second components can be administered as a pre-mixed combination (such as Oxytrex, currently being tested in clinical trials), or can be administered separately.

An opioid antagonist can be a partial agonist-antagonist or a narcotic antagonist. Examples of partial agonist-antagonists include, but are not limited to: butorphanol (Stadol), nalbuphine (Nubain), noscapine, and pentazocine (Talwin). Examples of pure narcotic antagonists include, but are not limited to: nadide (Enzopride), nalmefene, nalorphine (Nalline), naloxone, and naltrexone (ReVia).

The anti-opiopathic active agents employed in the present invention are commercially available or can be readily syntheszed. Thebaine and derivatives and analogues thereof can be synthesized by the methods disclosed by U.S. Patent Nos. 6,136,817 and 6,365,742. 14-Hydroxydihydro-morphinones, including naloxonazine, naloxazone, naloxone, naltrexazone, naltrexonazine, naltrexone, oxymorphazone, oxymorphone, oxymorphonazine, and analogues thereof can be synthesized by the methods disclosed by U.S. Patent No. 4,803,208. Morphine derivatives and analogues thereof can be synthesized by the methods disclosed by U.S. Patent Nos. 6,150,524 and 6,476,044. Opioids and opioid antagonists include the compounds disclosed by U.S. Patent Nos. 4,816,586 and 5,352,680, and U.S. Patent Application Publication No. US 2001/0047005.

Preferred for use as the anti-opiopathic active agent(s) in the present invention are: morphine, codeine, thebaine, papaverine, noscapine, hydromorphone, metapon, oxymorphone, levorphanol, hydrocodone, oxycodone, tramadol, nalorphine, naloxone, naltrexone, meperidine and congeners, methadone and congeners, levorphanol and congeners, phenazocine, propoxyphene, ethoheptazine, or a pharmaceutically or veterinarily acceptable salt thereof (immediate or sustained release, particularly sustained release). Particularly preferred are morphine, codeine, hydromorphone, hydrocodone, oxycodone, naloxone, naltrexone, or a pharmaceutically or veterinarily acceptable salt thereof. More particularly preferred are morphine, oxycodone or a pharmaceutically or veterinarily acceptable salt thereof. Most preferred is oxycodone hydrochloride sustained release.

There appears to exist a fairly well established bias against the use of opiates. Such bias extends to physicians and their patients, to veterinarians and their patients' owners. This negative bias is believed attributable to the inconvenience (paperwork) associated with prescribing opiates and concerns about addiction, theft, diversion and side effects (e.g., respiratory depression and particularly intoxication). Surprisingly, such customary side effects of opiate administration have not manifested in the instant subject group. In fact, contrary to concerns about respiratory depression and intoxication, these subjects have experienced improvements in respiration, energy, attentiveness and mobility. Addiction and concern thereabout are not expected to be issues in the treatment of opiopathies because the active anti-opiopathic agent supplies that which the patient's body is lacking, not something new that the body has learned to crave (providing a return too rather than an escape from normal). Moreover, it can be said that any patient who requires medication for relief from a chronic ailment is "addicted" to that medication in the sense of needing it to exist comfortably; however, prejudices have not arisen against other such medications or the patients who use them. Having identified an effective treatment for a series of ailments previously thought incurable, it is believed that physicians, veterinarians and their patients-will become more willing to consider the use of opiates, particularly where the alternatives are less effective or ineffective. Diversion is an issue that can be dealt with by various approaches known in the art, including some new approaches as described in greater detail below with respect to certain veterinary formulations. The elimination of opiate-associated paperwork for prescribing physicians and veterinarians, however, is beyond the scope of the present invention.

### PHARMACEUTICAL/VETERINARY FORMULATIONS AND MODES OF ADMINISTRATION

The present description also encompasses a formulation (pharmaceutical or veterinary) including a therapeutically effective amount of an anti-opiopathic active agent and optionally a carrier. Similarly, the description provides a kit incorporating such a formulation and instructions for its administration to treat an opiopathy. Also encompassed is the use of an anti-opiopathic active agent in the manufacture of a formulation for the treatment of an opiopathy. For example, an opioid compound can be employed in the manufacture of a medicament for use in treating paresis/paralysis, for treating pseudo-atrophy, and/or for treating opiopathic pain. The formulations, modes of administration, methods of use and manufacture are applicable to any of the above-referenced ailments, can include a mixture of two or more anti-opiopathic drugs, and can be for immediate or sustained release.

The pharmaceutical compositions can be administered by any suitable route including but not limited to: oral, rectal; nasal, topical (e.g., transdermal, aerosol, buccal, and sub-lingual), parenteral (e.g., subcutaneous, intramuscular, intravenous, intraperitoneal, intrathecal, and intracranial), or by inhalation (e.g., by nebulization, propellant atomizer or propellant inhaler). The preferred route of administration will depend on many variables (e.g., age, condition of the patient, concurrent diseases, formulations available for delivery) and the judgment of the prescribing physician or veterinarian.

Depending on the mode of administration, the pharmaceutical compositions can be in the form of a solid, semi-solid, or liquid. Examples include but are not limited to tablets (e.g., as in US patent 5,656,295), suppositories, bioerodable implants (e.g., ceramics such as in US patent 6,972,130), chewable veterinary formulations (e.g., as in US patent 5,780,046), pet foods (e.g., as in US patents 6,716,448 and 6,866,862), powders, liquids, suspensions, creams, ointments, lotions and the like, preferably in unit dosage form for single administration of a precise dosage. In addition to a carrier, the pharmaceutical formulation can Include other pharmaceutical agents, adjuvants, diluents, buffers, and the like. Actual methods of preparing such dosage forms are well known or will be apparent to those skilled in the art. For example, see: Remington: The Science and Practice of Pharmacy, Nineteenth Ed. (Easton, Pa.: Mack Publishing Company, 1995).

As previously discussed, one concern in expanding the use of opiates to a broader group of indications is that increased use of these drugs will lead to increased abuse. In that regard, one particular concern is the potential for diversion of anti-opiopathic active agents intended for veterinary use to abuse by humans. It is, therefore, another aspect of the present invention to provide a veterinarily acceptable formulation having characteristics rendering it less suitable (or completely unsuitable) for human consumption or diversion. Such formulation characteristics can be accomplished by inclusion of one or more "detractants," e.g., an odor, flavor, texture or other ingredient, or manufacture in a form that while acceptable, for example to dogs, would be unacceptable (repulsive) to human beings. Such veterinary formulations can be made chewable (such as the heartworm medicine sold under the mark "Heartguard"). The anti-opiopathic active agent can even be incorporated into a slowly erodable chew toy such as a synthetic bone (such as the breath fresheners sold under the mark "Greenies"). This approach takes advantage of the differential sensitivities between the species, dogs and cats generally being attracted by a variety of strong odors, flavors and/or textures that are generally unpalatable to human beings. In that regard, it is further envisioned that suitable veterinary formulations can be made to include one or more detractants that could otherwise be viewed as contaminants in a formulation intended for humans, such as hair, sand, insect parts or even feces (e.g., rodent, sheep or feline), provided that such detractants are rendered non-harmful via sterilization or similar processes; it is envisioned that the inclusion of such ingredients would be prominently displayed on the packaging to further dissuade diversion.

Both veterinary and pharmaceutical formulations can include additional ingredients, processing and/or packaging to render them tamper-resistant, to dissuade extraction and separation of the active agent(s) and thereby dissuade diversion or abuse. For example, such formulations can include exciplents that maintain the effectiveness of the active agent, such that its extraction and/or separation of the active agent from one or more such ingredients of the formulation would render the agent unpalatable, less active or completely inactive. Alternatively (or additionally), detractants can be added to these formulations to render them unsuitable for diversion.

### DOSAGE

The amount of active agent administered will be dependent on the particular drug selected, the age and general condition of the subject being treated, the severity of the subject's condition, the dosing regimen and the judgment of the prescribing physician or veterinarian. While daily dosing regimens can involve the administration of as many as eight doses, it is generally preferred that the number of doses be kept to a minimum for example to facilitate patient compliance. Moreover, the longer acting sustained-release dosage forms maintain a more consistent plasma concentration of the anti-opiopathic active agent, better simulating normal endogenous opioid concentrations and avoiding peaks and troughs that could give rise to periods of euphoria or craving. Generally, a daily dose of an active agent when administered ranges from about 0.1 mg to 200 mg, preferably about 1.0 to 100 mg, and more preferably about 5.0 to 50.0 mg, depending on the bioavailability and half-life of the anti-opiopathic active agent via the chosen route of administration; this will typically be consistent with the dosages recommended in the package inserts and information accompanying commercially available pharmaceuticals.. The dosing regimen can be modulated in order to achieve the desired effect.

The lowest effective dosage is the least amount of the pharmaceutical formulation sufficient to effect treatment. The lowest effective dose of an anti-opiopathic active agent used to control the presenting symptoms in the studies underlying the present invention was, oxycodone immediate release, in suspension, 3 mg administered every 12 hours, to a 47 lb subject. The highest tolerated dosage is the maximum amount of the pharmaceutical formulation to effect treatment without the occurrence of adverse side effect(s) outweighing the benefit received. Generally, the highest tolerated daily dose of OxyContin has been in the range of about 40 to 60 mg. The highest tolerated dose of an opioid formulation used in the studies underlying the present invention was 120 mg/day of OxyContin, administered as two (40 mg) tabs given every a.m., and one (40 mg) tab given every p.m.

For an animal subject weighing in the range of 60 to 80 pounds, such as a dog, a starting dose of OxyContin can be 5-10 mg given every 12 hours. A starting dose of morphine sulfate extended release for such a subject can be 7.5 to 15 mg given every 12 hours; for a cat 1.0 mg every 12 hours; for a horse 50 mg every 12 hours (route of administration adjusted conventionally for equine delivery). The dose of medication is adjusted according to the weight and need of a subject, to ameliorate the presenting symptoms. One of ordinary skill in the art will have the experience and means to determine the adjustment needed. Typically, if the symptoms have not started to resolve after about 2 to 14 days (depending on the neuropathic sign or symptom involved)of treatment (or if a subject starts to show the symptoms of drug tolerance) the dose is elevated by a factor ranging from 1.25 to 2.0 times the original dosage (preferably 1.5 times the original dose, e.g., 10 mg twice daily is increased to 15 mg twice daily) escalating on about a weekly basis until the opiopathic symptoms are effectively treated at a well-tolerated dosage. If the subject's highest tolerated dosage is reached, treatment should be discontinued, optionally re-commencing treatment by substituting a different anti-opiopathic active agent or formulation.

### EXAMPLES

The following examples serve to more fully describe the manner of using the above-described invention, as well as to set forth the best modes contemplated for carrying out various aspects of the invention. It is understood that these examples in no way serve to limit the true scope of this invention, but rather are presented for illustrative purposes.

Examples 1 - 8 illustrate the use of selected opiates for the treatment of specified neurologic/neurogenic disorders. These examples support the following:
(1) Pharmaceutical formulations from the group of drugs known as "opioids" are effective as a treatment for the neurologic/neurogenic symptoms associated with the disorders including lingual, pharyngeal, laryngeal, esophageal, urinary bladder sphincter, lumbar and lumbo-sacral spine, and pelvis and pelvic limb paresis/paralysis.
(2) When a subject's neurologic/neurogenic symptoms have been ameliorated by the use of a specific pharmaceutical formulation of opioid, substitution of a different, but equivalent pharmaceutical formulation of opioid does not guarantee continued successful treatment of the same symptoms (as in Example 4, where resuming administration of the initial agent successfully re-established treatment). In other subjects (as in Examples 7 and 8) continued successful treatment has been demonstrated after substitutions between sustained and immediate release formulations and between naturally occurring and semi-synthetic opioids.
(3) Different subjects with similar presenting neurologic/neurogenic disorders do not respond the same when treated with the same pharmaceutical formulations of opioids, in the same manner (as in Examples 2 and 3). As further illustrated in Example 3, successful treatment can be accomplished by consideration of the particular subject and symptoms, and adjusting the active agent, the dose and/or the formulation employed.

### Example 1

The initial patient in the studies underlying the present invention was a 10 year old, spayed female, very large dog; she was suffering from a number of problems common to older dogs. Since she was a puppy, she had suffered from "neurogenic urinary bladder sphincter incontinence," a condition that caused her to consistently leak urine (down her legs and in the area where she slept). This diagnosis had been confirmed by the Urology Department at the University of California at Davis, along with the fact that no viable treatment existed; she needed constant care to keep this area of her body as well as every place where she rested or slept clean and sanitary.

She was hypothyroid and receiving thyroid replacement medication. She also suffered from advanced bilateral hip dysplasia with osteoarthritis and degenerative joint disease. For this, she took an anti-arthritic medication every day, which appeared to provide some relief from the pain. She had started developing neurologic weakness with loss of muscle mass over her back, across her pelvis and down both rear legs over a period of about one to two years, making it very difficult for her to sit, stand, or walk without falling.

It was becoming increasingly difficult for her to breathe. She was diagnosed as suffering from "recurrent laryngeal nerve paralysis," the prognosis for which was to expect increasing difficulty until she reached the point where she would die from suffocation unless life-sparing surgery was performed to literally tie open the windpipe. I had a heart to heart talk with her owner when her condition had worsened to the point where a decision would soon need to be made, choosing between surgery to tie back her arytenoids, putting her to sleep or allowing her to die by suffocation.

A few Saturdays later I was reviewing answering machine messages and had received a frantic call from the owner saying "No, no, I'm not going to let her die this way." I called the house immediately and asked if he had gotten her to the emergency clinic. His reply surprised me. He apologized for alarming me because it had been a false alarm; he was sitting with her and she was OK. Knowing how tenuous her condition had been, I asked him to tell me what had happened. They had started out on their walk as usual when she began to choke and gasp; she seemed to be suffocating. In a true panic he had reached into his pocket and gave her two of his pain pills, hoping they would knock her out so she could suffocate in peace. He sat waiting with her head in his lap. After about twenty or so minutes she sat back up, and with his help she stood up and they walked home.

I asked what he had given her. He replied that it was a type of strong morphine called OxyContin. The use of a strong morphine raised concerns that she might still be suffocating (at the time, it was accepted practice to administer morphine to a suffocating frantic dog in heart failure, because it calmed the dog, slowed down the heart, improved oxygen availability and usage, and allowed the dog to become more stable until an examination could be completed and other medications administered). I arranged to meet him and his dog a half hour later. Just as he had said, she was calm and not suffocating. When I walked her briskly down the block, it appeared that not only was she not suffocating, she had no obstructive breathing sounds or symptoms. I instructed the owner to continue the same medication at 12 hour intervals until I could obtain more information.

I was unable to find any explanation for the reversal of her condition, and instructed the owner to continue the medication for as long as it continued to work. Every day when they walked past my clinic I went out to greet the two of them. As far as I could tell she was having no trouble breathing. About one week later she didn't seem to have the strong stagnant urine smell that had always accompanied her, and appeared more dry in the perivulvar area. By the end of the second week, she was walking without difficulty and appeared to have developed more muscle over her back and down each of her back legs. She continued this way for many months until she developed lung cancer and was eventually humanely euthanized.

### Example 2

About three days after the beginning of the events described in Example 1, a very frantic dog owner came running into my clinic carrying his Rhodesian Ridgeback. The dog was obviously gasping for air, shivering and unable to stand. She was diagnosed as suffering from acute laryngeal nerve paralysis, but was in such severe shock and hypothermia that she could not survive the emergency surgery needed to open her airway. Because this patient was probably going to die without treatment, it was decided that an attempt would be made to treat the condition by administering OxyContin and appropriate emergency care: IV catheter, fluids, warming procedures and the like.

After providing the above-described initial emergency treatment, a history was obtained from the owner. This was an outdoor dog. As she had aged, she had progressively experienced difficulty in breathing. She had also become progressively incontinent (this had not become a significant concern as she was an outside dog). Her ability to stand and walk had also deteriorated over the past few years. When her blood work came back she was clearly hypothyroid (for which thyroid replacement medication was prescribed).

Within 4 hours of being carried in to the clinic, this dog walked out on her own, breathing normally. A prescription was provided for twice-daily OxyContin and for thyroid replacement medication. She survived on this treatment for a considerable period of time until her death by euthanasia following a gastrointestinal crisis.

### Examples 3 - 8

In the period following the events described in Examples 1 and 2, treatment has been provided in over thirty-five cases of neuropathic disease involving paresis/paralysis of the tongue, larynx, pharynx, esophagus, urinary bladder sphincter, muscles of the lumbar and lumbo-sacral spine and of the pelvis and pelvic limbs. Having made the observations discussed in the above examples, these subsequent cases have been followed in the nature of a prospective study. After obtaining a detailed history each prospective subject is given a thorough physical and a neurological exam. Any non-opiopathic ailments are first treated traditionally and only upon treatment of such other ailments are the subjects considered eligible for treatment of any remaining neuropathic ailments using opioids (except where a critical patient presents as opiopathic suffocation, in which instances anti-opiopathic treatment has been immediately provided). Every subject in the study is videotaped before receiving treatment with an anti-opiopathic active agent, and videotaped at least once more during the study. Representative examples are provided below in Examples 3 to 8.

### Example 3

Subject: "JS," an 11 year old spayed, female, Standard Poodle (dog).

History and Examination: "JS's" symptoms included general body weakness, difficulty swallowing, difficult, noisy breathing, reflux of gastric acid into her esophagus, regurgitation of gastric acid from the esophagus into her oral and nasal cavities, with accompanying oral and nasal discharges. Visible wasting of the muscles over her lumbar and lumbo-sacral spine, and pelvis and pelvic limbs made it difficult to rise from a sitting position or walk without stumbling. She also exhibited an uncontrolled leaking of urine.

Diagnosis: UROS and OPS including: EP, LaP, LiP, PhP, LLSP, PPLP, NUBSP. JS's condition was so unstable at presentation that it took 72 hours to sort out and address all of her secondary medical problems. At this point only the above-described underlying, polyneuropathic syndrome remained.

Treatment: Sustained release oxycodone hydrochloride (OxyContin^{®}) - 1/8 of a 10 mg tablet every 12 hours.

Follow up: Within 2-3 hours following initiation of treatment, many of the neuropathic symptoms began to subside. Initially, she appeared more alert and interested in her surroundings. Shortly thereafter, the volume and strength of her respiration began to improve; as it did, most if not all of the obstructed laryngeal sounds seemed to subside. When asked to go outside for a walk, JS (who previously was too weak to stand) stood up, shook herself as if shaking water from her coat, and walked briskly towards the clinic's front door. When outside, she squatted, supporting her weight easily, urinated, stood back up and trotted back to the clinic door. She was sent home with the same medication, dose and dosing interval; her owners were instructed to call daily with progress reports. The following day, JS's owners reported that her condition had deteriorated almost as quickly, overnight, as it had improved the day before.

Examination: JS appeared very tired and reluctant to move or obey even simple commands such as heal or stand. Her head was hanging and the newly found interest in her surroundings had all but disappeared. Her heart rate, which had been between 120-140 bpm the prior day, was only 60-80 bpm at rest. Her respiratory rate, which had been markedly elevated the prior day, was now very depressed. Notwithstanding the foregoing, JS was still breathing without any of the obstructive sounds symptomatic of her presenting pharyngeal or laryngeal neuromyopathy.

Treatment: Medication was discontinued. JS' owners kept her stable and reported her vital signs daily.

Follow up: After 48 hours JS' cardiac and respiratory rates began to rise and her other symptoms that initially appeared to indicate a relapse, began to resolve. It was determined that JS was extremely sensitive to opiates and had experienced drug-induced depression as the result of an opioid overdose.

Treatment: After several attempts to adjust JS' medication dosages (hydrocodone IR, immediate release oxycodone hydrochloride (Roxycodone^{®} 20 mg/ml syrup), 1 drop every 24 hours.

Follow up: JS' symptoms of general body weakness, lingual, pharyngeal, laryngeal, esophageal, urinary bladder sphincter, lumbar and lumbo-sacral, pelvis and pelvic limb paresis/paralysis have resolved.

Conclusion: JS is believed to suffer from Myasthenia Gravis. A very low dose of immediate release oxycodone hydrochloride facilitated the dosage adjustment necessary and provided treatment. Other subjects suspected of suffering from Myasthenia Gravis should be started on low doses and immediate release formulations.

### Example 4

Subject: "ML," a 13 1/2 year old, spayed, female, large breed canine cross (dog)., suffered with a neurologic/neurogenic syndrome consisting of the following disorders: lingual paresis/paralysis, pharyngeal paresis/paralysis, laryngeal paresis/paralysis, urinary bladder sphincter paresis/paralysis, lumbar and lumbo-sacral spine paresis/paralysis, and pelvis and pelvic limb paresis/paralysis.

History and Examination: ML's symptoms included continual panting, dryness of the tongue and mouth, difficulty swallowing, choking, gagging and coughing, aspiratory difficulty accompanied by moist obstructive airway sounds, snoring, and progressive rear leg weakness especially noticeable because of the muscle atrophy seen over her lumbar and lumbo-sacral spine, pelvis and pelvic limbs. ML's owners had also noticed a problem with leaking of urine over the previous few years.

Diagnosis: UROS and OPS including: LaP, LiP, PhP, LSP, PLP and UBSP.

Treatment: Sustained release oxycodone hydrochloride (OxyContin^{®}) - one 10 mg tablet, every 12 hours.

Follow up: Within the first six hours following the initiation of treatment, ML's lingual, pharyngeal and laryngeal symptoms had all but abated. One week following the initiation of treatment, ML's urinary tract incontinence began to recede. By the 3rd week following the initiation of treatment, most of the muscle mass/tone had returned to the lumbar and lumbo-sacral spine, pelvis and pelvic limbs. This treatment regimen was continued for several months and continued to ameliorate all of ML's presenting symptoms.

Treatment change: Because of cost issues, the ML's owner elected to change her medication to an equivalent amount of the sustained release opioid agonist, Morphine Sulfate E.R., one 15 mg tablet every 12 hours.

Follow up: After one week on the new medication, all of the previous symptoms of ML's polyneuropathic syndrome had returned. She was again choking, gagging and coughing, having trouble swallowing, and showing symptoms of respiratory distress, especially when stressed or when exercising. Urine staining was noted in areas where she had been resting or sleeping. There was an almost complete loss of muscle mass/tone over her lumbar and lumbo-sacral spine, and down her pelvis and pelvic limbs, which accompanied the return of weakness and lack of coordination in these areas.

Treatment change: The owner was instructed to discontinue the Morphine Sulfate E.R. and to immediately resume administration of the previously prescribed dose of OxyContin^{®}.

Follow up: Twenty-four hours after resumption, the owner reported complete return of the laryngeal, pharyngeal, and lingual function. Over the next two weeks the function and muscling of the lumbar and lumbo-sacral spine, pelvis and pelvic limbs returned, as did the patency of the urinary bladder sphincter.

Conclusion: Sustained release oxycodone hydrochloride provided treatment for ML's UROS. Sustained release Morphine Sulfate E.R. proved ineffective for ML, precipitating a return of UROS with a surprisingly fast loss of lumbar and lumbo-sacral spin and pelvis and pelvic limb muscle mass/tone, which was equally surprisingly restored upon resumption of the initial treatment.

### Example 5

Subject: "KP," a three year old, neutered, male, Siberian Husky (dog).

Diagnosis: An inherited form of laryngeal paresis/paralysis.

Treatment: A high dose of sustained release oxycodone hydrochloride (OxyContin^{®}) - 40 mg am and 60 mg pm.

Follow up: The treatment has been effective in ameliorating the laryngeal paresis/paralysis.

Conclusion: Anti-opiopathic drug therapy provides treatment for individual neuromyopathies.

### Example 6

Subject: "PJ," a seventeen year old, neutered, male, Belgium Shepard (dog).

Diagnosis: UROS and OPS including: LaP, LiP, PhP, LSP, PLP and UBSP.

Treatment: Sustained release oxycodone hydrochloride (OxyContin^{®}) - one 10 mg tablet, every 12 hours.

Follow up: The treatment has been effective in eliminating or reducing these neuromyopathic symptoms of UROS and OPS.

Conclusion: With an otherwise healthy body, advanced age had no bearing on effective dosage levels.

### Example 7

Subject: "MG," a thirteen year old, neutered, male, mid-sized, Terrier cross (dog).

Diagnosis: UROS and OPS including: LaP, LiP, PhP and PLP.

Treatment: Initially, Morphine Sulfate E. R. (½ of a 15 mg tablet, every 12 hours) per the request of MG's owner out of concern about use of stronger medication.

Follow up: Dose escalation provided only minimal advancement toward treatment, and eventually signs of drug toxicity began to appear.

Treatment change: Sustained release oxycodone hydrochloride (OxyContin^{®}) - one 10 mg tablet, every 12 hours.

Follow up: The treatment has been effective in reducing these neuromyopathic symptoms of UROS and OPS.

Conclusion: Morphine sulfate E.R. was successfully replaced by OxyContin, which would have been the correct initial treatment absent the owner's request.

### Example 8

Subject: "LG," a fourteen year old, spayed, female, Golden Retriever (dog). about 80 pounds.

History: Had developed epilepsy during childbirth and continued seizures thereafter. Was being treated with Phenobarbital and potassium bromide.

Diagnosis: UROS and OPS including: LaP, LiP, PhP, PPLP.

Treatment: Initially, Morphine Sulfate I. R. suspension (20 mg/ml, 5-10 drops every 12 hours).

Follow up: The low initial doses were minimally effective for a short period of time, after which dose escalation became necessary every 2-3 days to sustain even such minimal treatment. Eventually it was decided to change to a stronger opioid in a sustained release formulation.

Treatment change: Sustained release oxycodone hydrochloride (OxyContin^{®})-½ 10 mg tablet, every 12 hours.

Follow up: The treatment has been effective in reducing these neuromyopathic symptoms of UROS and OPS.

Conclusion: It was not necessary to use immediate release morphine sulfate to avoid cross-reactivity with anticonvulsants. Oxycodone hydrochloride extended release can be employed as a first line of treatment for established presenting symptoms of UROS and OPS, even with concomitant anticonvulsant medication.

### Example 9

### Post-study Independent Analysis

Following participation in the study, for example, as described in Examples 3-9, the owners of participating dogs were asked to complete a questionnaire seeking information on their pet's symptoms before and after receiving anti-opiopathic treatment. Each of the following symptoms was rated on a scale of 0 to 10 (where 0 represented no impairment and 10 represented severe impairment): continuous panting, obstructive breathing, snoring, swallowing difficulty, choking or gagging, leaking urine, fecal incontinence, difficulty standing on front legs, difficulty standing on back legs, difficulty walking, body strength, and mental depression.

A detailed independent statistical analysis was performed using the responses. Significant reductions of severity were demonstrated in the sampled criteria (p<0.05 calculated using the Wilcoxon signed rank test). Table 1 displays the median scores as reported and Table 2 displays the results of statistical analysis of the data using the Wilcoxon signed rank test.

**Table 1**

| **Medians** | | | |
|---|---|---|---|
| **Symptom** | **n** | **Score Before** | **Score After** |
| Continuous Panting | 19 | 9.0 | 2.0 |
| Obstructive Breathing | 18 | 8.0 | 1.0 |
| Snoring | 14 | 5.5 | 1.5 |
| Swallowing Difficulty | 12 | 4.0 | 0.0 |
| Choking or Gagging | 14 | 5.5 | 0.5 |
| Leaking Urine | 17 | 8.0 | 1.0 |
| Fecal Incontinence | 13 | 5.0 | 0.0 |
| Difficulty Standing Up Front Legs | 17 | 5.0 | 1.0 |
| Difficulty Standing Up Back Legs | 21 | 8.0 | 2.0 |
| Difficulty Walking | 21 | 8.0 | 2.0 |
| Body Strength* | 19 | 7.0 | 3.0 |
| Mental Depression | 16 | 5.5 | 2.0 |
| n is the number of patients on which data was available both before and after treatment. | | | |

**Table 2**

| **Statistical Results** | | | | |
|---|---|---|---|---|
| **Symptom** | **n** | **Estimated Decrease** | **95% Conf. Interval** | **p-value** |
| Continuous Panting | 19 | -7.0 | (-8.0, -6.0) | 0.00031 |
| Obstructive Breathing | 18 | -7.5 | (-8.5, -6.0) | 0.00070 |
| Snoring | 14 | -5.0 | (-6.0, -3.0) | 0.00557 |
| Swallowing Difficulty | 12 | -4.5 | (-7.0, -3.5) | 0.01368 |
| Choking or Gagging | 14 | -5.5 | (-8.5, -3.0) | 0.00796 |
| Leaking Urine | 17 | -7.0 | (-8.5, -4.5) | 0.00148 |
| Fecal Incontinence | 13 | -5.5 | (-8.0, -3.0) | 0.03552 |
| Difficulty Standing Up Front Legs | 17 | -4.5 | (-7.0, -2.5) | 0.00667 |
| Difficulty Standing Up Back Legs | 21 | -6.0 | (-7.0, -4.5) | 0.00011 |
| Difficulty Walking | 21 | -5.0 | (-6.0, -3.5) | 0.00012 |
| Body Strength* | 19 | -2.5 | (-4.0, +0.5) | 0.11103 |
| Mental Depression | 16 | -3.5 | (-5.5, -1.0) | 0.01297 |
| n is the number of patients on which data was available both before and after treatment. Estimated decrease in score, 95% confidence interval, and p-value calculated using the Wilcoxon signed rank test. | | | | |

*The data reported for "body strength" may be subject to question because survey participants indicated that they were confused about how to interpret this term. "Total body strength" is considered a better term for use in subsequent surveys.

### Example 10

### Treatment of a Human Female Diagnosed as Having Multiple Sclerosis

Subject: "LB," a 54 year old, human female.

Diagnosis: A mild case of multiple sclerosis, specifically referred to as emotional incontinence. The diagnosis of this individual was performed by an independent neurologist, confirmed by spinal tap and MRI. A uterine biopsy and hormone levels confirmed no menopausal or peri-menopausal involvement.

Treatment: Hydrocodone/Tylenol 5/500 mg, 1 tablet every 8 hours. (This treatment was prescribed after consultation with the present inventor.)

Follow up: Effective treatment was provided, but did not last for the full 8 hours, resulting in returned symptoms prior to the time for the next dosage.

Treatment change: Repeat dosing accelerated when symptoms recurred before time for the next dose.

Conclusion: Dosage amount and frequency needs to be tailored to the requirements of the individual patient to obtain the best results. Patient should be considered for treatment with oxycodone hydrochloride, sustained release because this would eliminate breakthroughs and unnecessary administration of the anti-inflammatory with which the hydrocodone is formulated.

### Example 11

### Chewable Extended Release Tablet Formulation

**11A.** This example illustrates the preparation of a representative veterinary formulation for oral administration containing the anti-opiopathic active agent oxycodone hydrochloride and employing sand as a detractant. See Table 3.

Eudragit® RS 30D and Triacetin® are combined while passing through a 60 mesh screen, and mixed under low shear for approximately 5 minutes or until a uniform dispersion is observed. The oxycodone HCl, lactose (portion identified above as A) and povidone are placed into a fluid bed granulator/dryer (FBD) bowl, and the previously obtained suspension is sprayed onto the powder in the fluid bed. After spraying, the granulation is passed through a #12 screen if necessary to reduce lumps. The dry granulation is placed in a mixer, to which stearyl alcohol (previously melted at about 70°C) is added with mixing. The resulting waxed granulation is transferred to a fluid bed granulator/dryer or to trays, and allowed to cool to room temperature or below, followed (if necessary) by passage through a #12 screen.

**Table 3**

| Ingredient | Amount/Tablet | % (by wt) | Amount/ 1000 Tablet Batch |
|---|---|---|---|
| Oxycodone Hydrochloride | 10.0 mg | 0.5 % | 10.0 g |
| Eudragit ® RS 30D (solids) | 10.0 mg | 0.5 % | 10.0 g |
| Triacetin® | 2.0 mg | 0.1 % | 2.0 g |
| Lactose USP (spray dried) (A) | 70.0 mg | 3.5 % | 70.0 g |
| Povidone | 5.0 mg | 0.25 % | 5.0 g |
| Stearyl Alcohol | 25.0 mg | 1.25 % | 25.0 g |
| Sodium Starch Glycolate, NF (SSG) | 668.0 mg | 33.4 % | 668.0 g |
| Lactose USP (spray dried) (B) | 500.0 mg | 25.0 % | 500.0 g |
| Dessicated Liver | 300.0 mg | 15.0 % | 300.0 g |
| Sand (sterilized) | 200.0 mg | 10.0 % | 200.0 g |
| Dried Yeast | 65.0 mg | 3.25 % | 65.0 g |
| Aluminum Stearate | 45.0 mg | 2.25 % | 45.0 g |
| Fumaric Acid | 100.0 mg | 5.0 % | 100.0 g |
| Total: | 2,000.0 mg | 100.0 % | 2,000 g |

Sodium starch glycolate, lactose (portion identified above as B), fumaric acid, desiccated liver, sand and dried yeast are placed in a mixer and blended until uniform. A. portion (approximately 10%) of the resulting flavored mixture is removed to another mixer, combined with about 66.6% of the aluminum stearate, and mixed to uniformity. To this is added the remaining 90% of the flavored mixture, the fumaric acid and the waxed granulation first obtained above, followed by blending to an even distribution. The resulting mixture can then be slugged medium hard and sized through a rotary granulator using a #10 screen and the remaining 33.4% of the aluminum stearate added with mixing. The mixture thus obtained is compressed into tablet form. The resulting tablets can be given whole or broken into smaller sections for delivering a lower or incrementally higher dosage. The amounts of carriers can also be increased or lowered to adjust the size and volume of the chewable tablets.

**11B.** Other anti-opiopathic active agents (e.g., morphine sulfate, 15 mg), detractants (e.g., bug parts and/or sterilized cat feces) and/or excipients can be substituted in preparation of the formulations of this example.

### Example 12

### Packaged Veterinary Product

**12A.** A sufficient quantity (e.g., 60 units) of a veterinary formulation, such as a chewable, extended release tablet of Example 11, is sealed in one or more a blister packs marked for twice-daily administration. The blister packs are placed in a package (e.g., a box or a card) together with a printed package insert, labeled (e.g., "Canine Oxycodone Hydrochloride Liver Snaps, 10 mg, WARNING - NOT FOR HUMAN CONSUMPTION - CONTAINS SAND") and sealed (e.g., with tamper-evident shrink-wrap plastic). The sealed packages are stored under security measures appropriate for a controlled substance.

**12B.** The packaged veterinary product of the present example can include stronger, more graphic warning messages, e.g., "WARNING - CONTAINS CAT SHIT" or "WARNING - CONTAINS INGREDIENTS YOUR DOG WILL LOVE BUT YOU WILL HATE."

## Claims

1. A formulation for veterinary use that is adapted to dissuade misuse by humans comprising an opiate, a veterinarily accepted excipient and an ingredient that is suitable for administration to a non-human animal but unsuitable for administration to a human.

2. The formulation for veterinary use of Claim 1 wherein said ingredient comprises an odor, flavor or texture.

3. The formulation for veterinary use of Claim 1 wherein said ingredient is selected from the group consisting of hair, sand, insect parts and feces.

4. The formulation for veterinary use of Claim 1 wherein said ingredient is used to manufacture said formulation in a dosage form that is unsuitable for human consumption.

5. A veterinary product comprising a formulation for veterinary use according to Claim 1 and having packaging prominently labeled to highlight the presence of said ingredient.

6. The formulation for veterinary use of Claim 1 wherein said ingredient is repulsive to a human.

## Patentansprüche

1. Formulierung zur Anwendung am Tier, die angepasst ist um einen Missbrauch durch Menschen zu vermeiden, umfassend ein Opiat, ein für Tiere akzeptablen Trägerstoff und einen Inhaltsstoff, der für die Verabreichung an ein nicht-menschliches Tier geeignet ist, nicht hingegen für die Verabreichung an den Menschen geeignet ist.

2. Formulierung zur Anwendung am Tier nach Anspruch 1, wobei der Inhaltsstoff einen Geruch, Geschmack oder eine Textur umfasst.

3. Formulierung zur Anwendung am Tier nach Anspruch 1, wobei der Inhaltsstoff ausgewählt ist aus der Gruppe bestehend aus Haaren, Sand, Insektenteilen und Kot.

4. Formulierung zur Anwendung am Tier nach Anspruch 1, wobei der Inhaltsstoff verwendet wird, um die Formulierung in einer Darreichungsform herzustellen, die für den menschlichen Verzehr ungeeignet ist.

5. Veterinärprodukt umfassend eine Formulierung zur Anwendung am Tier gemäß Anspruch 1 mit einer auffälligen Verpackungskennzeichnung, die deutlich auf die Anwesenheit des Inhaltsstoffs hinweist.

6. Formulierung zur Anwendung am Tier nach Anspruch 1, wobei der Inhaltsstoff für den Menschen abstoßend ist.

## Revendications

1. Formulation destinée à un usage vétérinaire qui est adaptée pour dissuader un mésusage par des humains, comprenant un opiacé, un excipient acceptable d'un point de vue vétérinaire et un ingrédient qui est approprié pour une administration à un animal non humain mais est inapproprié pour une administration à un humain.

2. Formulation destinée à un usage vétérinaire selon la revendication 1, dans laquelle ledit ingrédient comprend une odeur, une saveur ou une texture.

3. Formulation destinée à un usage vétérinaire selon la revendication 1, dans laquelle ledit ingrédient est sélectionné dans le groupe consistant en des poils, du sable, des parties d'insecte et des selles.

4. Formulation destinée à un usage vétérinaire selon la revendication 1, dans laquelle ledit ingrédient est utilisé pour fabriquer ladite formulation sous une forme posologique qui est inappropriée pour une consommation humaine.

5. Produit vétérinaire comprenant une formulation destinée à un usage vétérinaire selon la revendication 1 et comportant un emballage principalement étiqueté pour mettre en évidence la présence dudit ingrédient.

6. Formulation destinée à un usage vétérinaire selon la revendication 1, dans laquelle ledit ingrédient est répulsif pour un humain.
